# EUROPEAN PATENT SPECIFICATION

(11) **EP 2 331 485 B1**
(45) Date of publication and mention of the grant of the patent: **07.12.2016**
(21) Application number: 09818338.7
(22) Date of filing: 29.09.2009
(51) Int. Cl.: C07C 2/00, B01J 29/00, C07C 2/66

(54) **PROCESS FOR ETHYLBENZENE PRODUCTION**
VERFAHREN ZUR HERSTELLUNG VON ETHYLBENZEN
PROCÉDÉ DE PRODUCTION D'ÉTHYLBENZÈNE

(30) Priority: 28.09.2009 US 568007; 30.09.2008 US 101610 P
(43) Date of publication of application: 15.06.2011
(73) Proprietor: Fina Technology, Inc., Houston, TX 77267-4412 (US)
(72) Inventor: BUTLER, James, R., League City, TX 77573 (US)
(74) Representative: Raboin, Jean-Christophe
(86) International application number: PCT/US2009/058674
(87) International publication number: WO 2010/039661

(56) References cited:
- US-A- 4 101 596
- US-A- 5 227 558
- US-A- 5 600 050
- US-A- 5 723 710
- US-A- 5 907 073
- US-A- 6 034 291
- US-A1- 2004 015 030
- US-A1- 2005 143 612
- US-A1- 2006 116 539
- US-A1- 2008 058 567
- US-A1- 2008 058 568
- HONGLIN WANG ET.AL.: "Surface acidity of H-beta and its catalytic activity for alkylation of benzene with propylene", CATALYSIS LETTERS, vol. 76, no. 3-4, 2001, pages 225-229, XP002722416,

## Description

### CROSS-REFERENCE TO RELATED APPLICATIONS

The present invention claims priority to provisional application number 61/101610 filed on September 30,2008.

### FIELD

Embodiments of the present invention generally relate to alkylation of aromatic compounds.

### BACKGROUND

Alkylation reactions generally involve contacting a first aromatic compound with an alkylation agent in the presence of a catalyst to form a second aromatic compound. One important alkylation reaction is the reaction of benzene with ethylene in the production of ethylbenzene (US 5,600,050)

. The ethylbenzene can then be dehydrogenated to form
styrene.

Wang et al., Catalysis Letters, vol. 76, no. 3-4, 2001, 225-229 discloses the alkylation of benzene with propylene.

Catalyst life is an important consideration in alkylation reactions. There are the costs related to the catalyst itself, such as the unit cost of the catalyst, the useful life of the catalyst, the ability to regenerate used catalyst, and the cost of disposing of used catalyst. There are also the costs related to shutting down an alkylation reactor to replace the catalyst and/or to regenerate the catalyst bed, which includes labor, materials, and loss of productivity.

Catalyst deactivation can tend to reduce the level of conversion, the level of selectivity, or both, each which can result in an undesirable loss of process efficiency. There can be various reasons for deactivation of alkylation catalysts. These can include the plugging of catalyst surfaces, such as by coke or tars, which can be referred to as carbonization; the physical breakdown of the catalyst structure; and the loss of promoters or additives from the catalyst. Depending upon the catalyst and the various operating parameters that are used, one or more of these mechanisms may apply.

Another cause of catalyst deactivation can be the result of poisons present in an input stream to the alkylation system, for example amine or ammonia compounds. The poisons can react with components of the catalyst leading to deactivation of the component or a restriction in accessing the component within the catalyst structure. The poisons can further act to reduce yields and increase costs. Therefore, a need exists to develop an alkylation system that is capable of reducing alkylation catalyst deactivation or a method of managing alkylation catalyst deactivation in an effective manner.

In view of the above, it would be desirable to have an effective method to produce ethylbenzene in commercial quantities via a catalytic alkylation reaction. It would further be desirable if the method was robust and did not experience frequent disruptions due to process interruptions for catalyst regeneration or replacement.

### SUMMARY

Embodiments of the present invention include a method of producing commercial quantities of ethylbenzene by the catalytic alkylation reaction of benzene and ethylene.

Embodiments of the present invention include a method of producing alkylaromatics by the alkylation of an aromatic and an alkylating agent, the method involving providing at least one reaction zone containing H-beta zeolite catalyst into which a feed stream comprising an aromatic and an alkylating agent is introduced. At least a portion of the aromatic is reacted under alkylation conditions to produce an alkylaromatic. A first product stream containing alkylaromatic can then be removed. The aromatic can be benzene, the alkylating agent can be ethylene, and the alkylaromatic can be ethylbenzene. The alkylaromatic production can be least 0.5 million pounds per day and can be between at least 0.5 million pounds per day and 10 million pounds per day.

The at least one reaction zone can include at least one preliminary alkylation reactor and at least one primary alkylation reactor. The at least one preliminary alkylation reactor can contain H-beta zeolite catalyst in a quantity of between at least 5,000 pounds to 50,000 pounds. One or more of the at least one preliminary alkylation reactor and at least one primary alkylation reactor can contain a mixed catalyst that includes the H-beta zeolite catalyst in addition to at least one other catalyst. In an embodiment the primary alkylation reactor experiences a reduced rate of catalyst deactivation when the preliminary alkylation reactor containing H-beta zeolite catalyst is in service. In an embodiment the primary alkylation reactor experiences no catalyst deactivation when the preliminary alkylation reactor containing H-beta zeolite catalyst is in service.

The amount of H-beta catalyst can be between at least 5,000 pounds to 50,000 pounds and can be in a preliminary alkylation system, which can have a run time of at least 6 months, or at least 9 months, or at least 12 months, or at least 18 months prior to regeneration. The H-beta zeolite catalyst in the preliminary alkylation system can be regenerated in-situ. The preliminary alkylation system can be bypassed for catalyst regeneration without taking the at least one primary alkylation reactor out of service.

### BRIEF DESCRIPTION OF DRAWINGS

Figure 1 is a schematic block diagram of an embodiment of an alkylation/transalkylation process.
Figure 2 is a schematic block diagram of an embodiment of an alkylation/transalkylation process that includes a preliminary alkylation step.
Figure 3 is a schematic illustration of a parallel reactor system that can be used for a preliminary alkylation step.
Figure 4 illustrates one embodiment of an alkylation reactor with a plurality of catalyst beds.
Figure 5 is a graph of the percent temperature rise data obtained in an Example of the present invention.

### DETAILED DESCRIPTION

The present application is directed to a method of producing an alkylaromatic by the alkylation of an aromatic with an alkylating agent, the method comprising: providing at least one reaction zone containing a H-beta zeolite catalyst, wherein the at least one reaction zone comprises at least one preliminary alkylation reactor and at least one primary alkylation reactor downstream of the preliminary alkylation reactor, wherein the H-beta zeolite catalyst is contained in the preliminary alkylation reactor and a catalyst other than the H-beta zeolite catalyst is contained in the primary alkylation reactor; introducing a feed stream comprising an aromatic and an alkylating agent to the reaction zone, wherein the feed stream further includes catalyst poisons, which are nitrogen compounds; and reacting at least a portion of the aromatic under alkylation conditions to produce an alkylaromatic.

Aromatic conversion processes carried out over molecular sieve catalysts are well known in the chemical industry. Alkylation reactions of aromatics, such as benzene, to produce a variety of alkyl-benzene derivatives, such as ethylbenzene, are quite common.

Embodiments of the present invention generally relate an alkylation system adapted to minimize process upsets due to alkylation catalyst deactivation and the resulting catalyst regeneration or replacement. In one embodiment of the invention, commercial quantities of H-beta catalyst are used within an alkylation process to produce commercial quantities of ethylbenzene from benzene and ethylene. The process can include one or more fixed catalyst beds of H-beta that can be regenerated either in-situ or ex-situ without significant disruptions to the commercial alkylation production rates.

As used herein commercial quantities of an H-beta Alkylation catalyst means a quantity of from 3,000 pounds to 50,000 pounds or more of catalyst in use as an alkylation system within an alkylation process, such as for ethylbenzene production. The H-beta alkylation catalyst can be used as a preliminary alkylation system within an alkylation process for ethylbenzene production. The preliminary Alkylation system can be an initial bed or beds in a multi-bed reactor, or can be an initial reactor or group of reactors in a multi-reactor Alkylation process, for example. In embodiments of the invention where an H-beta Alkylation catalyst is utilized for both the preliminary alkylation system and the primary Alkylation system, the catalyst quantity for the total process may range up to 100,000 pounds or more. As used herein commercial quantities of ethylbenzene from the Alkylation process can range from an average daily production of 0.5 million pounds up to 10.0 million pounds of ethylbenzene or more.

Zeolite beta catalysts are suitable for use in the present invention and are well known in the art. Zeolite beta catalysts typically have a silica/alumina molar ratio (expressed as SiO₂/Al₂O₃) of from about 10 to about 300, or about 15 to about 75, for example. In one embodiment, the zeolite beta may have a low sodium content, e.g., less than about 0.2 wt% expressed as Na₂O, or less than about 0.06 wt%, for example. The sodium content may be reduced by any method known to one skilled in the art, such as through ion exchange, for example. Zeolite beta catalysts are characterized by having a high surface area of at least 400 m²/g based upon the crystalline form without any regard to supplemental components such as binders. In one embodiment, the zeolite beta may have a surface area of at least 600 m²/g, The formation of zeolite beta catalysts is further described in U.S Patent No. 3,308,069 to Wadlinger et al and U.S Patent No. 4,642,226 to Calvert et al,

An H-beta type zeolite catalyst has the characteristic of having hydrogen as its nominal cation form. Within one particular embodiment a commercially available H-beta catalyst from Zeolyst International with a commercial designation of Zeolyst CP 787 Zeolite H-Beta Extrudate is used in commercial quantities for the production of ethylbenzene by the alkylation reaction of benzene and ethylene.

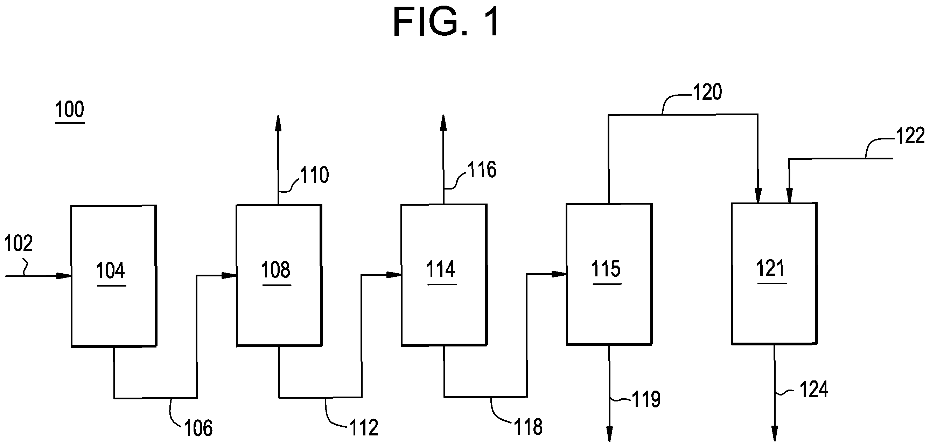

Figure 1 illustrates a schematic block diagram of an embodiment of an alkylation/transalkylation process 100. The process 100 generally includes supplying an input stream 102 (e.g., a first input stream) to an alkylation system 104 (e.g., a first alkylation system.) The alkylation system 104 is generally adapted to contact the input stream 102 with an alkylation catalyst to form an alkylation output stream 106 (e.g., a first output stream).

At least a portion of the alkylation output stream 106 passes to a first separation system 108. An overhead fraction is generally recovered from the first separation system 108 via line 110 while at least a portion of the bottoms fraction is passed via line 112 to a second separation system 114.

An overhead fraction is generally recovered from the second separation system 114 via line 116 while at least a portion of a bottoms fraction is passed via line 118 to a third separation system 115. A bottoms fraction is generally recovered from the third separation system 115 via line 119 while at least a portion of an overhead fraction is passed via line 120 to a transalkylation system 121. In addition to the overhead fraction 120, an additional input, such as additional aromatic compound, is generally supplied to the transalkylation system 121 via line 122 and contacts the transalkyation catalyst, forming a transalkylation output 124.

Although not shown herein, the process stream flow may be modified based on unit optimization. For example, at least a portion of any overhead fraction may be recycled as input to any other system within the process. Also, additional process equipment, such as heat exchangers, may be employed throughout the processes described herein and placement of the process equipment can be as is generally known to one skilled in the art. Further, while described in terms of primary components, the streams indicated may include any additional components as known to one skilled in the art.

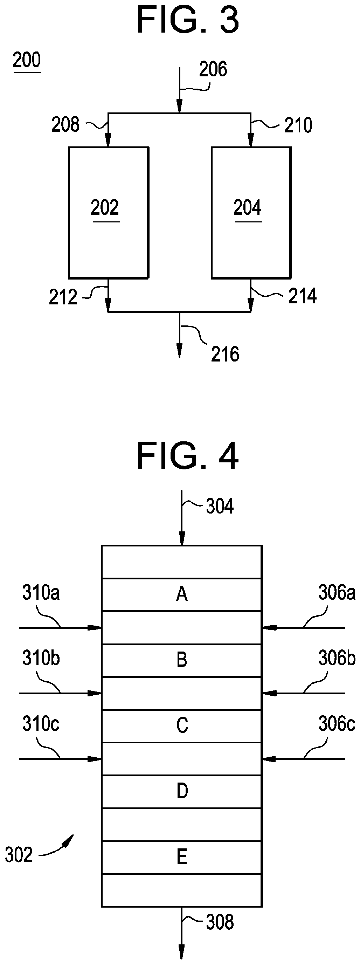

The input stream 102 generally includes an aromatic compound and an alkylating agent. The aromatic compound may include substituted or unsubstituted aromatic compounds. The aromatic compound may include hydrocarbons, such as benzene, for example. If present, the substituents on the aromatic compounds may be independently selected from alkyl, aryl, alkaryl, alkoxy, aryloxy, cycloalkyl, halide and/or other groups that do not interfere with the alkylation reaction, for example. The input stream and alkylating agent 102 can be input at multiple locations as shown in Figure 4.

The alkylating agent may include olefins such as ethylene or propylene, for example. In one embodiment, the aromatic compound is benzene and the alkylating agent is ethylene, which react to form a product that includes ethylbenzene as a significant component, for example.

In addition to the aromatic compound and the alkylating agent, the input stream 102 may further include other compounds in minor amounts (e.g., sometimes referred to as poisons or inactive compounds). Poisons can be nitrogen components such as ammonia, amine compounds, or nitriles, for example. These poisons can be in quantities in the parts-per-billion (ppb) range, but can have significant effect on the catalyst performance and reduce its useful life. In one embodiment, the input stream 102 includes up to 100 ppb or more of poisons. In one embodiment, the input stream 102 includes poisons typically ranging from 10 ppb to 50 ppb. In one embodiment, the poison content typically averages from 20 ppb to 40 ppb.

Inactive compounds, which can be referred to as inert compounds, such as C₆ to C₈ aliphatic compounds may also be present. In one embodiment, the input stream 102 includes less than about 5% of such compounds or less than about 1%, for example.

The alkylation system 104 can include a plurality of multi-stage reaction vessels. In one embodiment, the multi-stage reaction vessels can include a plurality of operably connected catalyst beds, such beds containing an alkylation catalyst, such as shown in Figure 4 for example. Such reaction vessels are generally liquid phase reactors operated at reactor temperatures and pressures sufficient to maintain the alkylation reaction in the liquid phase, i.e., the aromatic compound is in the liquid phase. Such temperatures and pressures are generally determined by individual process parameters. For example, the reaction vessel temperature may be from 65°C to 300°C, or from 200°C to 280°C, for example. The reaction vessel pressure may be any suitable pressure in which the alkylation reaction can take place in the liquid phase, such as from 300 psig to 1,200 psig, for example.

In one embodiment, the space velocity of the reaction vessel within the alkylation system 104 is from 10 liquid hourly space velocity (LHSV) per bed to 200 LHSV per bed, based on the aromatic feed rate. In alternate embodiments, the LHSV can range from 10 to 100, or from 10 to 50, or from 10 to 25 per bed. For the alkylation process overall, including all of the alkylation beds of the preliminary alkylation reactor or reactors and the primary alkylation reactor or reactors, the space velocity can range from 1 LHSV to 20 LHSV.

The alkylation output 106 generally includes a second aromatic compound. In one embodiment, the second aromatic compound includes ethylbenzene, for example.

A first separation system 108 may include any process or combination of processes known to one skilled in the art for the separation of aromatic compounds. For example, the first separation system 108 may include one or more distillation columns (not shown,) either in series or in parallel. The number of such columns may depend on the volume of the alkylation output 106 passing through.

The overhead fraction 110 from the first separation system 108 generally includes the first aromatic compound, such as benzene, for example.

The bottoms fraction 112 from the first separation system 108 generally includes the second aromatic compound, such as ethylbenzene, for example.

A second separation system 114 may include any process known to one skilled in the art, for example, one or more distillation columns (not shown), either in series or in parallel.

The overhead fraction 116 from the second separation system 114 generally includes the second aromatic compound, such as ethylbenzene, which may be recovered and used for any suitable purpose, such as the production of styrene, for example.

The bottoms fraction 118 from the second separation system 114 generally includes heavier aromatic compounds, such as polyethylbenzene, cumene and/or butylbenzene, for example.

A third separation system 115 generally includes any process known to one skilled in the art, for example, one or more distillation columns (not shown), either in series or in parallel.

In a specific embodiment, the overhead fraction 120 from the third separation system 115 may include diethylbenzene and triethylbenzene, for example. The bottoms fraction 119 (e.g., heavies) may be recovered from the third separation system 115 for further processing and recovery (not shown).

The transalkylation system 121 generally includes one or more reaction vessels having a transalkylation catalyst disposed therein. The reaction vessels may include any reaction vessel, combination of reaction vessels and/or number of reaction vessels (either in parallel or in series) known to one skilled in the art.

A transalkylation output 124 generally includes the second aromatic compound, for example, ethylbenzene. The transalkylation output 124 can be sent to one of the separation systems, such as the second separation system 114, for separation of the components of the transalkylation output 124.

In one embodiment, the transalkylation system 121 is operated under liquid phase conditions. For example, the transalkylation system 121 may be operated at a temperature of from about 65°C to about 290°C and a pressure of about 800 psig or less.

In a specific embodiment, the input stream 102 includes benzene and ethylene. The benzene may be supplied from a variety of sources, such as for example; a fresh benzene source and/or a variety of recycle sources. As used herein, the term "fresh benzene source" refers to a source including at least about 95 wt% benzene, at least about 98 wt% benzene or at least about 99 wt% benzene, for example. In one embodiment, the molar ratio of benzene to ethylene may be from about 1:1 to about 30:1, or from about 1:1 to about 20:1, for the total alkylation process including all of the alkylation beds, for example. The molar ratio of benzene to ethylene for individual alkylation beds can range from 10:1 to 100:1, for example.

In a specific embodiment, benzene is recovered through line 110 and recycled (not shown) as input to the alkylation system 104, while ethylbenzene and/or polyalkylated benzenes are recovered via line 112.

As previously discussed, the alkylation system 104 generally includes an alkylation catalyst. The input stream 102, e.g., benzene/ethylene, contacts the alkylation catalyst during the alkylation reaction to form the alkylation output 106, e.g., ethylbenzene.

Unfortunately, alkylation catalyst systems generally experience deactivation requiring either regeneration or replacement. Additionally, alkylation processes generally require periodic maintenance. Both circumstances generally produce disruptions for liquid phase alkylation processes. The deactivation results from a number of factors. One of those factors is that poisons present in the input stream 102, such as nitrogen, sulfur and/or oxygen containing impurities, either naturally occurring or a result of a prior process, may reduce the activity of the alkylation catalyst.

Embodiments of the invention provide a process wherein continuous production during catalyst regeneration and maintenance may be achieved. For example, one reactor may be taken off-line for regeneration of the catalyst, either by in-situ or ex-situ methods, while the remaining reactor may remain on-line for production. The determination of when such regeneration will be required can depend on specific system conditions, but is generally a predetermined set point (e.g., catalyst productivity, temperature, or time).

If in-situ regeneration is not possible, when removing the catalyst from the reactor for regeneration, it may be possible to replace the catalyst and place the reactor on-line while the removed/deactivated catalyst is regenerated. In such an embodiment, the cost of replacing the catalyst can be large and therefore it is beneficial that such catalyst should have a long life before regeneration. Embodiments of the invention may provide an alkylation system capable of extended catalyst life and extended production runs.

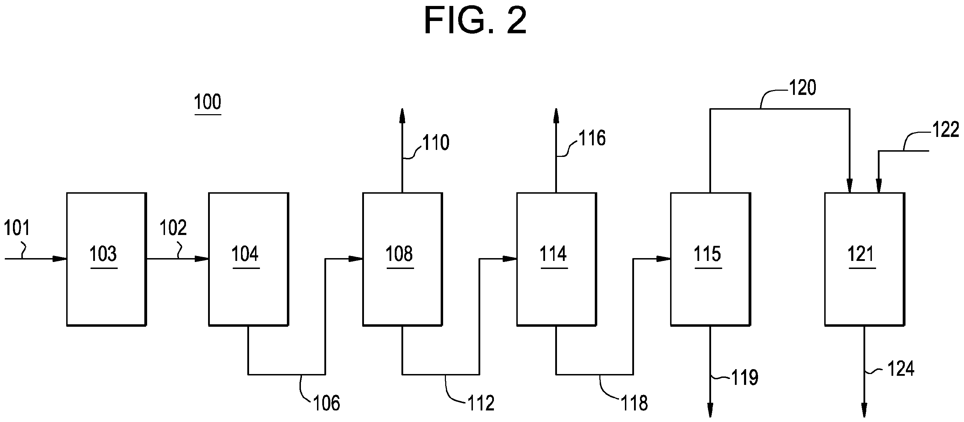

Referring to Figure 2, the alkylation/transalkylation system 100 may further include a preliminary alkylation system 103. The preliminary alkylation system 103 may be maintained at alkylation conditions, for example. The preliminary alkylation input stream 101 may be passed through the preliminary alkylation system 103 prior to entry into the alkylation system 104 to reduce the level of poisons in the input stream 102, for example. In one embodiment, the level of poisons is reduced by at least 10%, or at least 25% or at least 40% or at least 60% or at least 80%, for example. For example, the preliminary alkylation system 103 may be used as a sacrificial system, thereby reducing the amount of poisons contacting the alkylation catalyst in the alkylation system 104 and reducing the frequency of regeneration needed of the alkylation catalyst in the alkylation system 104.

In one embodiment the preliminary alkylation input stream 101 comprises the entire benzene feed to the process and a portion of the ethylene feed to the process. This feed passes through the preliminary alkylation system 103 that contains the zeolite beta catalyst prior to entry into the alkylation system 104 to reduce the level of poisons contacting the alkylation catalyst in the alkylation system 104. The output stream 102 from the preliminary alkylation system 103 can include unreacted benzene and ethylbenzene produced from the preliminary alkylation system 103. Additional ethylene can be added to the alkylation system 104 (not shown in Figure 2) to react with the unreacted benzene. In this embodiment the preliminary alkylation system 103 can reduce the level of poisons in the benzene and that portion of the ethylene feed that is added to the process preliminary alkylation input stream 101. Ethylene that is added after the preliminary alkylation system 103, such as to the alkylation system 104, would not have a reduction in the level of poisons from the preliminary alkylation system 103.

The preliminary alkylation system 103 may be operated under liquid phase conditions. For example, the preliminary alkylation system 103 may be operated at a temperature of from about 100°C to about 300°C, or from 200°C to about 280°C, and a pressure to ensure liquid phase conditions, such as from about 300 psig to about 1200 psig.

The preliminary alkylation system 103 generally includes a preliminary catalyst (not shown) disposed therein. The alkylation catalyst, transalkylation catalyst and/or the preliminary catalyst may be the same or different. In general, such catalysts are selected from molecular sieve catalysts, such as zeolite beta catalysts, for example.

As a result of the level of poisons present in the preliminary alkylation input 101, the preliminary catalyst in the preliminary alkylation system 103 may become deactivated, requiring regeneration and/or replacement. For example, the preliminary catalyst may experience deactivation more rapidly than the alkylation catalyst.

Embodiments of the invention can utilize a H-beta zeolite catalyst in the preliminary alkylation system 103. In addition the alkylation reaction may also utilize such H-beta catalyst. Embodiments can include the preliminary alkylation system having a mixed catalyst load that includes a H-beta zeolite catalyst and one or more other catalyst. The mixed catalyst load can, for example, be a layering of the various catalysts, either with or without a barrier or separation between them, or alternately can include a physical mixing where the various catalysts are in contact with each other. Embodiments can include the alkylation system having a mixed catalyst load that includes a H-beta zeolite catalyst and one or more other catalyst. The mixed catalyst load can, for example, be a layering of the various catalysts, either with or without a barrier or separation between them, or alternately can include a physical mixing where the various catalysts are in contact with each other.

When regeneration of any catalyst within the system is desired, the regeneration procedure generally includes processing the deactivated catalyst at high temperatures, although the regeneration may include any regeneration procedure known to one skilled in the art.

Once a reactor is taken off-line, the catalyst disposed therein may be purged. Off-stream reactor purging may be performed by contacting the catalyst in the off-line reactor with a purging stream, which may include any suitable inert gas (e.g., nitrogen), for example. The off-stream reactor purging conditions are generally determined by individual process parameters and are generally known to one skilled in the art.

The catalyst may then undergo regeneration. The regeneration conditions may be any conditions that are effective for at least partially reactivating the catalyst and are generally known to one skilled in the art. For example, regeneration may include heating the alkylation catalyst to a temperature or a series of temperatures, such as a regeneration temperature of from about 200°C to about 500°C above the purging or alkylation reaction temperature, for example.

In one embodiment, the alkylation catalyst is heated to a first temperature (e.g., 400°C) with a gas containing nitrogen and 2 mol% or less oxygen, for example, for a time sufficient to provide an output stream having an oxygen content of about 0.1 mol%. The regeneration conditions will generally be controlled by the alkylation system restrictions and/or operating permit requirements that can regulate conditions, such as the permissible oxygen content that can be sent to flare for emission controls. The alkylation catalyst may then be heated to a second temperature (e.g., 500°C) for a time sufficient to provide an output stream having a certain oxygen content. The catalyst may further be held at the second temperature for a period of time, or at a third temperature that is greater than the second temperature, for example. Upon catalyst regeneration, the reactor is allowed to cool and can then be made ready to be placed on-line for continued production.

Figure 3 illustrates a non-limiting embodiment of an alkylation system 200, which can be a preliminary alkylation system. The alkylation system 200 shown includes a plurality of alkylation reactors, such as two alkylation reactors 202 and 204, operating in parallel. One or both alkylation reactors 202 and 204, which may be the same type of reaction vessel, or, in certain embodiments, may be different types of reaction vessels, may be placed in service at the same time. For example, only one alkylation reactor may be on line while the other is undergoing maintenance, such as catalyst regeneration. In one embodiment, the alkylation system 200 is configured so that the input stream is split to supply approximately the same input to each alkylation reactor 202 and 204. However, such flow rates will be determined by each individual system.

This mode of operation (e.g., multiple parallel reactors) may involve operation of the individual reactors at relatively lower space velocities for prolonged periods of time with periodic relatively short periods of operation at enhanced, relatively higher space velocities when one reactor is taken off-stream. By way of example, during normal operation of the system 200, with both reactors 202 and 204 on-line, the input 206 stream may be supplied to each reactor (e.g., via lines 208 and 210) to provide a reduced space velocity. The output 216 stream may be the combined flow from each reactor (e.g., via lines 212 and 214). When a reactor is taken off-line and the feed rate continues unabated, the space velocity for the remaining reactor may approximately double.

In a specific embodiment, one or more of the plurality of alkylation reactors may include a plurality of interconnected catalyst beds. The plurality of catalyst beds may include from 2 to 15 beds, or from 5 to 10 beds or, in specific embodiments, 5 or 8 beds, for example. Embodiments can include one or more catalyst beds having a mixed catalyst load that includes a H-beta zeolite catalyst and one or more other catalyst. The mixed catalyst load can, for example, be a layering of the various catalysts, either with or without a barrier or separation between them, or alternately can include a physical mixing where the various catalysts are in contact with each other.

Figure 4 illustrates a non-limiting embodiment of an alkylation reactor 302. The alkylation reactor 302 includes five series connected catalyst beds designated as beds A, B, C, D, and E. An input stream 304 (e.g., benzene/ethylene) is introduced to the reactor 302, passing through each of the catalyst beds to contact the alkylation catalyst and form the alkylation output 308. Additional alkylating agent may be supplied via lines 306a, 306b, and 306c to the interstage locations in the reactor 302. Additional aromatic compound may also be introduced to the interstage locations via lines 310a, 310b and 310c, for example.

### EXAMPLE

In Example 1 a process of making ethylbenzene using commercial quantities of a H-beta zeolite includes a preliminary alkylation system having a single reactor loaded with approximately 22,000 pounds of H-beta zeolite catalyst. The process further comprises a primary alkylation system after the preliminary alkylation system that contains catalyst other than the H-beta zeolite catalyst.

The feed stream to the process can contain impurities such as acetonitrile, ammonia, and/or amine compounds, for example, in quantities that range from 1 ppb to 100 ppb or more and can typically average from 20 ppb to 40 ppb. The preliminary alkylation system can remove impurities in the benzene feed and a portion of the ethylene feed to the process prior to the primary alkylation system. The H-beta catalyst is commercially available from Zeolyst International with a commercial designation of Zeolyst CP 787 Zeolite H-Beta Extrudate.

The benzene feed is added to the preliminary alkylation reactor at a rate of approximately 700,000 to 750,000 pounds per hour, passes through the preliminary alkylation reactor and then to the primary alkylation system. The benzene feed is equivalent to approximately 15 to 20 LHSV for the preliminary alkylation reactor.

Ethylene is added to both the preliminary alkylation reactor and to the primary alkylation system. Ethylene is added to the process in a benzene:ethylene molar ratio typically ranging from between 15:1 to 20:1 for the preliminary alkylation reactor and for each catalyst bed within the primary alkylation system. The process, including the preliminary alkylation reactor and the primary alkylation system, has an overall benzene:ethylene molar ratio typically ranging from between 2.7:1 to 3.7:1. Conversion of benzene to ethylbenzene in the preliminary alkylation reactor results in about 1.0 million pounds per day of the total ethylbenzene production. The process, including the preliminary alkylation reactor and the primary alkylation system, has an overall production rate of about 7.5 million pounds of ethylbenzene per day.

During Example 1 the primary alkylation reaction beds did not shown significant signs of deactivation, indicating that the preliminary bed is containing, reacting or deactivating the poisons that are present in the benzene feed.

Table 1 provides selected data obtained from Example 1. The data is presented as a percentage of the overall temperature rise in the preliminary alkylation reactor that has occurred at specific locations. Thermocouple #1 (TW #1) provides the temperature reading at a point approximately 11% into the length of the preliminary alkylation reactor catalyst bed and thereby can give an indication of the amount of reaction that has occurred in the first 11% of the bed, which represents about 2,400 pounds of catalyst. Thermocouple #2 (TW #2) is approximately 31% through the preliminary alkylation reactor catalyst bed, which represents about 6,800 pounds of catalyst, while thermocouple #3 (TW #3) is approximately 47% through the preliminary alkylation reactor catalyst bed, which represents about 10,300 pounds of catalyst, and thermocouple #4 (TW #4) is approximately 64% through the preliminary alkylation reactor catalyst bed, which represents about 14,100 pounds of catalyst. The data in Table 1 is not normalized to force a maximum percent rise to 100%. Values of over 100% can be due to temperature reading variations among the various instruments.

The temperature profiles of the preliminary alkylation reactor catalyst bed indicate where the catalytic reaction is occurring and the extent of catalyst deactivation along the length of the bed. As the catalyst deactivates and the active reaction zone proceeds down the length of the bed to catalyst that is active, the temperature rise profile can be observed to progress down the reactor. For example if the percent rise at TW #1 is 50%, then approximately 50% of the entire temperature rise throughout the preliminary catalyst bed is occurring within the first 11% of the bed. If later the percent rise at TW #1 value decreases to 20%, that would indicate that the catalyst in the first 11% of the catalyst bed has deactivated to an extent that only 20% of the temperature rise is occurring in the first 11% of the bed length while 80% of the rise is occurring after the first 11% of the catalyst bed length.

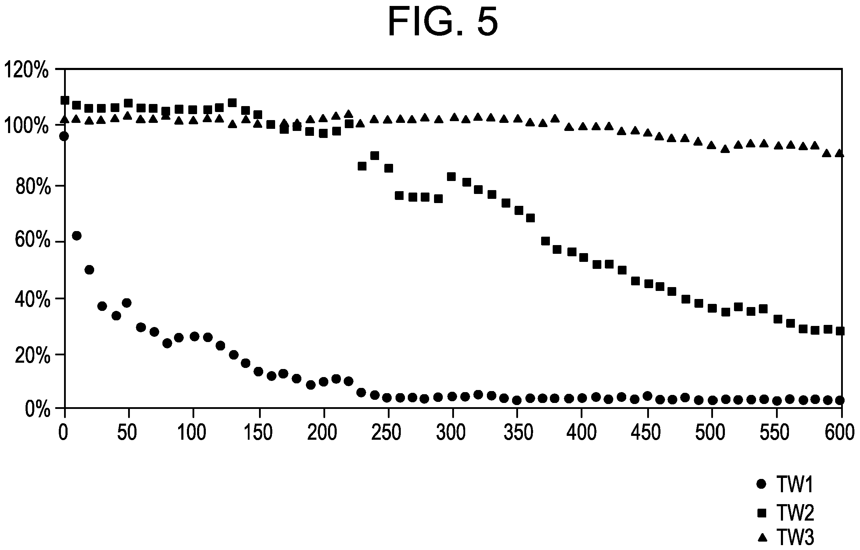

The preliminary alkylation reactor containing H-beta zeolite catalyst was in service for over 580 days without requiring regeneration. Figure 5 illustrates the temperature trend data for TW #1, TW #2 and TW #3 for the first 600 days of Example 1. The data points shown are the percent rise on approximately every 10 days. Figure 5 is only to illustrate the trends in the data of Table 1 and should not be taken to supersede Table 1 in any way. Upon 100 days service, the percent rise at TW # 1 (11% into the length of the reactor) had decreased from an initial 89% to around 25%, while the percent rise at TW #2 (31% into the length of the reactor) had not shown any appreciable decrease. Upon 200 days service, the percent rise at TW #1 had decreased to around 10%, while the percent rise at TW #2 had decreased to around 95%, while the percent rise at TW #3 (47% into the length of the reactor) had not shown any appreciable decrease. Upon 300 days service, the percent rise at TW #1 had decreased to around 5%, the percent rise at TW #2 had decreased to around 80%, while the percent rise at TW #3 had not shown any appreciable decrease. Upon 400 days service, the percent rise at TW #1 had decreased to less than 5%, the percent rise at TW #2 had decreased to around 55%, while the percent rise at TW #3 had just started to indicate a decrease. Upon 500 days service, the percent rise at TW #1 had decreased to around 2%, the percent rise at TW #2 had decreased to around 35%, the percent rise at TW #3 had decreased to around 94%, while the percent rise at TW #4 (64% into the length of the reactor) had not shown any appreciable decrease. Upon 600 days service, the percent rise at TW #1 was still around 2%, the percent rise at TW #2 had decreased to around 26%, the percent rise at TW #3 had decreased to around 90%, while the percent rise at TW #4 (64% into the length of the reactor) had still not shown any appreciable decrease. During Example 1 the rate of deactivation of the catalyst in the primary alkylation reactor located after the preliminary alkylation reactor was less than the rate of deactivation prior to having the preliminary alkylation reactor in service. There was a reduction of catalyst deactivation in the primary alkylation system, indicating the preliminary alkylation reactor was able to contain or react with poisons contained in the benzene feed such that they had a reduced effect on the catalyst in the primary alkylation system.

A total of about 1,360 million pounds of EB was produced by the process during the first 190 days of Example 1, of which 181 days had online production, with a deactivation of about ninety percent of the catalyst load in the preliminary alkylation reactor up to TW #1 and twenty percent of the catalyst load between TW#1 to TW #2, which equates to about 3,000 pounds of deactivated catalyst. This portion of Example 1 provided about 0.45 million pounds of EB production per pound of catalyst deactivation in the preliminary alkylation reactor, or alternatively had a deactivation of about 2.2 pounds of catalyst per million pounds of EB produced.

A total of about 2,625 million pounds of EB was produced by the process during the first 365 days of Example 1, of which 350 days had online production, with a deactivation of about 96% of the catalyst load in the preliminary alkylation reactor up to TW #1 and 30% of the catalyst load between TW#1 to TW #2, which equates to about 3,600 pounds of deactivated catalyst. The first 350 days of online production provided a deactivation of about 1.4 pounds of catalyst per million pounds of EB produced.

A total of about 4,350 million pounds of EB was produced by the process during the first 595 days of Example 1, of which 580 days had online production, with a deactivation of about 97% of the catalyst load in the preliminary alkylation reactor up to TW #1, 79% of the catalyst load between TW#1 to TW #2, and 15% of the catalyst load between TW#2 to TW #3, which equates to about 6,330 pounds of deactivated catalyst. The first 580 days of online production of Example 1 provided a deactivation of about 1.45 pounds of catalyst per million pounds of EB produced.

Referring to Figure 5, although the catalyst contained in the first 11% of the preliminary alkylation reactor bed had been in service for 580 days, it was still providing about 3% of the temperature rise across the preliminary alkylation reactor, indicating that it still had some activity. The decline curve for TW #2 is less steep than that of TW #1, indicating that the catalyst before the TW #1 location in the catalyst bed is reducing the effect of poisons present in the benzene feed on the catalyst down stream, thus extending its useful catalyst life. The decline in the TW #3 curve is less steep than that of TW #1 during its initial deactivation and is less steep than that of TW #2, further indicating that the catalyst before the TW #2 location in the catalyst bed is removing a significant amount of the poisons present in the feed, thus extending the useful catalyst life of the catalyst down stream.

An embodiment of the present invention involves a process of producing ethylbenzene from a high poison feed stream by the alkylation of benzene with ethylene utilizing an H-beta zeolite catalyst that has a catalyst deactivation rate that is no more than 30 pounds of catalyst per million pounds of EB produced. Additional embodiments include a process having a catalyst deactivation rate that is no more than 20 pounds of catalyst per million pounds of EB produced, no more than 10 pounds of catalyst per million pounds of EB produced, no more than 7.5 pounds of catalyst per million pounds of EB produced, no more than 5 pounds of catalyst per million pounds of EB produced, no more than 2.5 pounds of catalyst per million pounds of EB produced, no more than 2.0 pounds of catalyst per million pounds of EB produced, and no more than 1.5 pounds of catalyst per million pounds of EB produced.

**Table 1 Preliminary Alkylation Reactor Data**

| Date | Overall temp rise °F | TW #1 temp rise | TW #2 temp rise | TW #3 temp rise | TW #4 temp rise |
|---|---|---|---|---|---|
| 01/22/08 | 47.5 | 89% | 109% | 102% | 102% |
| 01/23/08 | 53.0 | 87% | 108% | 102% | 103% |
| 01/24/08 | 53.5 | 84% | 107% | 103% | 103% |
| 01/25/08 | 54.5 | 75% | 106% | 103% | 103% |
| 01/26/08 | 53.7 | 72% | 108% | 103% | 103% |
| 01/27/08 | 53.7 | 71% | 108% | 102% | 103% |
| 01/28/08 | 54.1 | 70% | 107% | 102% | 102% |
| 01/29/08 | 54.5 | 64% | 108% | 102% | 103% |
| 01/30/08 | 54.9 | 63% | 108% | 103% | 104% |
| 01/31/08 | 54.8 | 63% | 107% | 102% | 103% |
| 02/01/08 | 55.0 | 59% | 107% | 102% | 103% |
| 02/02/08 | 55.5 | 58% | 106% | 101% | 102% |
| 02/03/08 | 55.4 | 56% | 106% | 101% | 101% |
| 02/04/08 | 55.7 | 55% | 106% | 101% | 101% |
| 02/05/08 | 56.0 | 54% | 106% | 101% | 102% |
| 02/06/08 | 55.4 | 55% | 107% | 102% | 103% |
| 02/07/08 | 55.9 | 54% | 107% | 102% | 103% |
| 02/08/08 | 56.3 | 53% | 107% | 102% | 102% |
| 02/09/08 | 56.8 | 52% | 106% | 102% | 102% |
| 02/10/08 | 56.8 | 51% | 106% | 101% | 102% |
| 02/11/08 | 56.5 | 50% | 106% | 101% | 102% |
| 02/12/08 | 56.4 | 48% | 107% | 102% | 103% |
| 02/13/08 | 56.7 | 45% | 107% | 103% | 104% |
| 02/14/08 | 56.5 | 44% | 107% | 102% | 102% |
| 02/15/08 | 56.7 | 43% | 106% | 102% | 102% |
| 02/16/08 | 56.9 | 42% | 106% | 102% | 102% |
| 02/17/08 | 56.7 | 41% | 106% | 102% | 102% |
| 02/18/08 | 55.3 | 40% | 107% | 102% | 103% |
| 02/19/08 | 53.5 | 38% | 106% | 102% | 102% |
| 02/20/08 | 52.2 | 36% | 106% | 101% | 102% |
| 02/21/08 | 55.4 | 37% | 105% | 101% | 102% |
| 02/22/08 | 56.1 | 37% | 106% | 102% | 103% |
| 02/23/08 | 56.2 | 37% | 106% | 102% | 102% |
| 02/24/08 | 56.1 | 36% | 106% | 102% | 102% |
| 02/25/08 | 56.2 | 35% | 105% | 101% | 102% |
| 02/26/08 | 56.3 | 35% | 107% | 103% | 103% |
| 02/27/08 | 55.4 | 35% | 107% | 103% | 104% |
| 02/28/08 | 55.4 | 34% | 106% | 102% | 102% |
| 02/29/08 | 55.5 | 33% | 106% | 101% | 102% |
| 03/01/08 | 55.4 | 33% | 106% | 102% | 102% |
| 03/02/08 | 55.6 | 32% | 105% | 101% | 102% |
| 03/03/08 | 55.5 | 32% | 106% | 102% | 103% |
| 03/04/08 | 55.6 | 32% | 106% | 103% | 104% |
| 03/05/08 | 55.3 | 31% | 106% | 102% | 103% |
| 03/06/08 | 55.5 | 31% | 106% | 102% | 103% |
| 03/07/08 | 55.6 | 30% | 107% | 103% | 105% |
| 03/08/08 | 55.2 | 31% | 106% | 103% | 103% |
| 03/09/08 | 55.3 | 30% | 106% | 102% | 103% |
| 03/10/08 | 53.8 | 34% | 107% | 102% | 103% |
| 03/11/08 | 52.5 | 37% | 108% | 103% | 104% |
| 03/12/08 | 52.7 | 38% | 108% | 103% | 103% |
| 03/13/08 | 53.2 | 30% | 105% | 102% | 102% |
| 03/14/08 | 53.7 | 26% | 104% | 101% | 101% |
| 03/15/08 | 53.5 | 27% | 104% | 102% | 102% |
| 03/16/08 | 53.8 | 26% | 104% | 101% | 102% |
| 03/17/08 | 54.8 | 25% | 103% | 101% | 101% |
| 03/18/08 | 55.1 | 24% | 103% | 101% | 101% |
| 03/19/08 | 55.1 | 24% | 104% | 102% | 103% |
| 03/20/08 | 55.8 | 25% | 104% | 102% | 103% |
| 03/21/08 | 55.3 | 25% | 104% | 102% | 102% |
| 03/22/08 | 54.6 | 28% | 106% | 102% | 103% |
| 03/23/08 | 54.8 | 33% | 107% | 103% | 104% |
| 03/24/08 | 56.1 | 25% | 105% | 104% | 104% |
| 03/25/08 | 56.9 | 24% | 104% | 102% | 102% |
| 03/26/08 | 55.9 | 24% | 104% | 102% | 102% |
| 03/29/08 | 55.5 | 23% | 104% | 101% | 102% |
| 03/30/08 | 55.4 | 23% | 103% | 101% | 102% |
| 03/31/08 | 57.6 | 23% | 101% | 99% | 101% |
| 04/01/08 | 53.9 | 22% | 103% | 101% | 102% |
| 04/02/08 | 49.8 | 26% | 106% | 103% | 104% |
| 04/03/08 | 41.3 | 26% | 106% | 102% | 103% |
| 04/04/08 | 55.6 | 25% | 104% | 102% | 102% |
| 04/05/08 | 54.0 | 23% | 104% | 103% | 103% |
| 04/06/08 | 55.7 | 23% | 103% | 101% | 102% |
| 04/07/08 | 56.7 | 24% | 104% | 101% | 102% |
| 04/08/08 | 20.2 | 30% | 116% | 104% | 107% |
| 04/09/08 | 57.3 | 23% | 103% | 101% | 102% |
| 04/10/08 | 57.0 | 22% | 103% | 101% | 101% |
| 04/11/08 | 53.2 | 22% | 101% | 100% | 100% |
| 04/12/08 | 44.5 | 27% | 106% | 104% | 104% |
| 04/13/08 | 54.4 | 22% | 104% | 103% | 104% |
| 04/14/08 | 56.1 | 21% | 103% | 103% | 104% |
| 04/15/08 | 56.8 | 21% | 102% | 102% | 103% |
| 04/16/08 | 56.4 | 21% | 102% | 101% | 102% |
| 04/17/08 | 55.6 | 23% | 104% | 101% | 102% |
| 04/18/08 | 54.7 | 25% | 106% | 102% | 103% |
| 04/19/08 | 54.8 | 25% | 106% | 102% | 103% |
| 04/20/08 | 54.7 | 25% | 106% | 102% | 102% |
| 04/21/08 | 54.8 | 24% | 105% | 102% | 102% |
| 04/22/08 | 54.6 | 23% | 105% | 101% | 102% |
| 04/23/08 | 64.8 | 24% | 105% | 101% | 101% |
| 04/24/08 | 55.0 | 22% | 105% | 101% | 101% |
| 04/25/08 | 10.8 | 42% | 136% | 111% | 112% |
| 04/26/08 | 54.0 | 25% | 106% | 102% | 103% |
| 04/27/08 | 55.8 | 23% | 106% | 102% | 103% |
| 04/28/08 | 54.7 | 22% | 105% | 102% | 103% |
| 04/29/08 | 54.3 | 21% | 105% | 102% | 103% |
| 04/30/08 | 54.6 | 22% | 104% | 102% | 102% |
| 05/01/08 | 54.0 | 24% | 105% | 101% | 101% |
| 05/02/08 | 51.8 | 21% | 103% | 102% | 102% |
| 05/03/08 | 47.0 | 28% | 109% | 102% | 104% |
| 05/04/08 | 47.4 | 29% | 108% | 103% | 104% |
| 05/05/08 | 47.0 | 30% | 109% | 103% | 103% |
| 05/06/08 | 44.1 | 31% | 109% | 102% | 103% |
| 05/07/08 | 44.2 | 31% | 109% | 102% | 102% |
| 05/08/08 | 43.2 | 31% | 109% | 103% | 103% |
| 05/15/08 | 46.0 | 30% | 109% | 105% | 106% |
| 05/16/08 | 54.4 | 24% | 106% | 102% | 103% |
| 05/17/08 | 54.2 | 24% | 106% | 102% | 103% |
| 05/18/08 | 54.4 | 24% | 106% | 102% | 102% |
| 05/19/08 | 53.7 | 24% | 105% | 102% | 102% |
| 05/20/08 | 55.4 | 25% | 106% | 102% | 102% |
| 05/21/08 | 55.5 | 26% | 106% | 101% | 102% |
| 05/22/08 | 55.6 | 25% | 106% | 101% | 102% |
| 05/23/08 | 55.0 | 24% | 106% | 101% | 101% |
| 05/24/08 | 53.3 | 28% | 107% | 101% | 102% |
| 05/25/08 | 51.9 | 29% | 107% | 101% | 102% |
| 05/26/08 | 47.3 | 34% | 109% | 101% | 102% |
| 05/27/08 | 51.1 | 31% | 108% | 102% | 102% |
| 05/28/08 | 50.0 | 26% | 107% | 102% | 102% |
| 05/29/08 | 49.4 | 25% | 106% | 101% | 102% |
| 05/30/08 | 49.2 | 25% | 106% | 101% | 101% |
| 05/31/08 | 49.1 | 24% | 106% | 101% | 102% |
| 06/01/08 | 49.3 | 24% | 106% | 101% | 102% |
| 06/02/08 | 54.7 | 20% | 103% | 101% | 101% |
| 06/03/08 | 56.2 | 18% | 103% | 101% | 101% |
| 06/04/08 | 56.3 | 18% | 102% | 101% | 101% |
| 06/05/08 | 56.1 | 19% | 103% | 101% | 101% |
| 06/06/08 | 55.9 | 18% | 103% | 100% | 101% |
| 06/07/08 | 55.9 | 18% | 103% | 100% | 101% |
| 06/08/08 | 55.6 | 19% | 103% | 101% | 101% |
| 06/09/08 | 55.8 | 18% | 103% | 100% | 101% |
| 06/10/08 | 55.8 | 18% | 103% | 100% | 101% |
| 06/11/08 | 55.7 | 18% | 103% | 101% | 101% |
| 06/12/08 | 56.5 | 18% | 102% | 100% | 101% |
| 06/13/08 | 55.9 | 17% | 102% | 100% | 101% |
| 06/14/08 | 55.9 | 18% | 103% | 101% | 101% |
| 06/15/08 | 55.9 | 18% | 103% | 101% | 102% |
| 06/16/08 | 56.0 | 17% | 102% | 101% | 101% |
| 06/17/08 | 55.6 | 17% | 102% | 101% | 102% |
| 06/18/08 | 55.6 | 17% | 102% | 101% | 102% |
| 06/19/08 | 55.7 | 17% | 102% | 101% | 101% |
| 06/20/08 | 55.7 | 17% | 102% | 101% | 101% |
| 06/21/08 | 55.7 | 17% | 102% | 100% | 101% |
| 06/22/08 | 55.7 | 16% | 102% | 101% | 101% |
| 06/23/08 | 55.6 | 16% | 102% | 101% | 102% |
| 06/24/08 | 56.4 | 15% | 100% | 100% | 101% |
| 06/25/08 | 56.5 | 15% | 99% | 100% | 101% |
| 06/26/08 | 56.5 | 15% | 100% | 100% | 101% |
| 06/27/08 | 56.5 | 15% | 99% | 100% | 102% |
| 06/28/08 | 56.4 | 14% | 99% | 101% | 102% |
| 06/29/08 | 56.5 | 15% | 99% | 101% | 102% |
| 06/30/08 | 56.5 | 14% | 98% | 100% | 102% |
| 07/01/08 | 49.0 | 20% | 106% | 102% | 103% |
| 07/02/08 | 50.7 | 19% | 106% | 101% | 102% |
| 07/03/08 | 50.4 | 19% | 106% | 102% | 103% |
| 07/04/08 | 55.8 | 15% | 101% | 101% | 102% |
| 07/05/08 | 56.4 | 14% | 99% | 101% | 102% |
| 07/06/08 | 55.5 | 13% | 98% | 100% | 101% |
| 07/07/08 | 55.4 | 13% | 98% | 100% | 101% |
| 07/08/08 | 55.4 | 13% | 98% | 101% | 101% |
| 07/09/08 | 55.5 | 12% | 97% | 101% | 102% |
| 07/10/08 | 55.3 | 12% | 97% | 101% | 101% |
| 07/11/08 | 55.3 | 13% | 97% | 101% | 101% |
| 07/12/08 | 55.1 | 12% | 96% | 100% | 101% |
| 07/13/08 | 55.1 | 12% | 96% | 100% | 101% |
| 07/14/08 | 55.2 | 12% | 97% | 101% | 102% |
| 07/15/08 | 55.4 | 12% | 97% | 100% | 101% |
| 07/16/08 | 53.8 | 14% | 99% | 100% | 101% |
| 07/17/08 | 53.2 | 14% | 101% | 101% | 102% |
| 07/18/08 | 53.3 | 14% | 101% | 100% | 102% |
| 07/19/08 | 53.6 | 14% | 100% | 100% | 101% |
| 07/20/08 | 53.8 | 14% | 100% | 100% | 101% |
| 07/21/08 | 53.6 | 14% | 100% | 100% | 102% |
| 07/22/08 | 53.7 | 14% | 100% | 100% | 101% |
| 07/23/08 | 53.8 | 14% | 101% | 101% | 102% |
| 07/24/08 | 53.7 | 14% | 101% | 101% | 102% |
| 07/25/08 | 56.6 | 14% | 97% | 100% | 101% |
| 07/26/08 | 56.1 | 12% | 96% | 100% | 101% |
| 07/27/08 | 56.9 | 13% | 96% | 101% | 102% |
| 07/28/08 | 55.9 | 11% | 97% | 101% | 102% |
| 07/29/08 | 54.1 | 15% | 102% | 101% | 102% |
| 07/30/08 | 52.7 | 13% | 100% | 101% | 102% |
| 07/31/08 | 56.2 | 11% | 96% | 101% | 102% |
| 08/01/08 | 54.8 | 11% | 96% | 101% | 102% |
| 08/02/08 | 54.5 | 11% | 95% | 101% | 101% |
| 08/03/08 | 54.6 | 11% | 95% | 101% | 102% |
| 08/04/08 | 55.3 | 11% | 95% | 101% | 102% |
| 08/05/08 | 55.9 | 10% | 95% | 101% | 102% |
| 08/06/08 | 56.1 | 10% | 95% | 101% | 101% |
| 08/07/08 | 56.0 | 10% | 95% | 101% | 102% |
| 08/08/08 | 55.3 | 10% | 95% | 101% | 102% |
| 08/09/08 | 55.3 | 10% | 94% | 100% | 102% |
| 08/10/08 | 55.5 | 10% | 94% | 100% | 101% |
| 08/11/08 | 54.3 | 11% | 95% | 101% | 102% |
| 08/12/08 | 53.7 | 11% | 96% | 101% | 102% |
| 08/13/08 | 53.6 | 11% | 96% | 101% | 102% |
| 08/14/08 | 53.6 | 11% | 96% | 101% | 102% |
| 08/15/08 | 53.6 | 11% | 96% | 102% | 102% |
| 08/16/08 | 53.6 | 10% | 96% | 101% | 102% |
| 08/17/08 | 53.6 | 10% | 95% | 101% | 102% |
| 08/18/08 | 53.7 | 10% | 95% | 101% | 102% |
| 08/19/08 | 53.6 | 10% | 95% | 102% | 102% |
| 08/20/08 | 53.0 | 10% | 95% | 101% | 102% |
| 08/21/08 | 46.5 | 12% | 102% | 101% | 102% |
| 08/22/08 | 44.5 | 11% | 94% | 101% | 103% |
| 08/23/08 | 43.4 | 10% | 95% | 103% | 104% |
| 08/24/08 | 43.8 | 11% | 95% | 102% | 104% |
| 08/25/08 | 44.3 | 13% | 101% | 103% | 104% |
| 08/26/08 | 49.2 | 12% | 103% | 102% | 103% |
| 08/27/08 | 49.2 | 12% | 102% | 101% | 103% |
| 08/28/08 | 48.6 | 11% | 96% | 102% | 103% |
| 09/14/08 | 42.0 | 13% | 107% | 103% | 104% |
| 09/15/08 | 40.4 | 17% | 112% | 109% | 110% |
| 09/16/08 | 46.6 | 13% | 108% | 105% | 106% |
| 09/17/08 | 48.1 | 13% | 106% | 104% | 104% |
| 09/18/08 | 48.1 | 14% | 106% | 103% | 104% |
| 09/19/08 | 48.0 | 12% | 104% | 102% | 103% |
| 09/20/08 | 47.6 | 12% | 104% | 104% | 105% |
| 09/21/08 | 47.9 | 11% | 103% | 103% | 104% |
| 09/22/08 | 48.0 | 12% | 104% | 104% | 105% |
| 09/23/08 | 56.0 | 9% | 96% | 102% | 103% |
| 09/24/08 | 55.6 | 7% | 91% | 102% | 103% |
| 09/25/08 | 55.7 | 8% | 90% | 102% | 103% |
| 09/26/08 | 55.6 | 7% | 89% | 101% | 102% |
| 09/27/08 | 55.6 | 7% | 88% | 101% | 102% |
| 09/28/08 | 55.8 | 7% | 87% | 101% | 103% |
| 09/29/08 | 54.3 | 8% | 90% | 101% | 103% |
| 09/30/08 | 56.4 | 7% | 87% | 100% | 102% |
| 10/01/08 | 57.2 | 6% | 86% | 101% | 102% |
| 10/02/08 | 57.1 | 6% | 85% | 100% | 102% |
| 10/03/08 | 56.6 | 6% | 86% | 100% | 101% |
| 10/04/08 | 56.4 | 7% | 87% | 100% | 102% |
| 10/05/08 | 55.6 | 7% | 89% | 100% | 102% |
| 10/06/08 | 56.5 | 7% | 87% | 100% | 101% |
| 10/07/08 | 57.6 | 7% | 84% | 100% | 101% |
| 10/08/08 | 57.0 | 6% | 82% | 100% | 102% |
| 10/09/08 | 56.7 | 6% | 82% | 101% | 103% |
| 10/10/08 | 55.8 | 6% | 83% | 101% | 102% |
| 10/11/08 | 55.7 | 6% | 82% | 101% | 102% |
| 10/12/08 | 55.8 | 6% | 82% | 101% | 103% |
| 10/13/08 | 55.4 | 5% | 84% | 100% | 103% |
| 10/14/08 | 56.2 | 6% | 85% | 100% | 102% |
| 10/15/08 | 56.1 | 6% | 83% | 100% | 102% |
| 10/16/08 | 55.8 | 6% | 83% | 100% | 102% |
| 10/17/08 | 55.8 | 6% | 83% | 101% | 103% |
| 10/18/08 | 55.8 | 6% | 83% | 102% | 103% |
| 10/19/08 | 55.9 | 6% | 82% | 102% | 103% |
| 10/20/08 | 55.5 | 5% | 82% | 101% | 102% |
| 10/21/08 | 55.4 | 5% | 85% | 101% | 103% |
| 10/22/08 | 55.5 | 6% | 85% | 102% | 103% |
| 10/23/08 | 54.9 | 5% | 85% | 102% | 104% |
| 10/24/08 | 55.0 | 5% | 84% | 101% | 103% |
| 10/25/08 | 54.9 | 5% | 84% | 102% | 103% |
| 10/26/08 | 55.3 | 5% | 80% | 101% | 103% |
| 10/27/08 | 54.1 | 5% | 81% | 102% | 104% |
| 10/28/08 | 52.4 | 7% | 94% | 103% | 105% |
| 10/29/08 | 53.6 | 4% | 81% | 101% | 103% |
| 10/30/08 | 56.0 | 5% | 76% | 101% | 103% |
| 10/31/08 | 54.4 | 5% | 75% | 100% | 102% |
| 11/01/08 | 54.3 | 5% | 75% | 101% | 103% |
| 11/02/08 | 54.4 | 5% | 75% | 100% | 103% |
| 11/03/08 | 54.5 | 5% | 76% | 100% | 102% |
| 11/04/08 | 55.1 | 5% | 75% | 101% | 103% |
| 11/05/08 | 55.6 | 5% | 74% | 100% | 102% |
| 11/06/08 | 55.5 | 5% | 75% | 100% | 102% |
| 11/07/08 | 55.3 | 5% | 75% | 101% | 103% |
| 11/08/08 | 55.4 | 5% | 76% | 101% | 102% |
| 11/09/08 | 55.1 | 5% | 76% | 101% | 103% |
| 11/10/08 | 55.6 | 5% | 75% | 101% | 103% |
| 11/11/08 | 55.2 | 5% | 76% | 100% | 102% |
| 11/12/08 | 54.6 | 5% | 78% | 101% | 103% |
| 11/13/08 | 55.0 | 5% | 77% | 100% | 103% |
| 11/14/08 | 54.7 | 5% | 78% | 101% | 103% |
| 11/15/08 | 54.5 | 4% | 77% | 102% | 104% |
| 11/16/08 | 53.5 | 4% | 76% | 102% | 104% |
| 11/17/08 | 52.8 | 4% | 76% | 101% | 103% |
| 11/18/08 | 53.9 | 4% | 75% | 102% | 104% |
| 11/19/08 | 53.4 | 4% | 75% | 102% | 103% |
| 11/20/08 | 53.2 | 4% | 75% | 102% | 104% |
| 11/21/08 | 52.5 | 4% | 75% | 103% | 105% |
| 11/22/08 | 52.9 | 5% | 76% | 102% | 104% |
| 11/23/08 | 53.5 | 4% | 75% | 101% | 103% |
| 11/24/08 | 52.8 | 4% | 75% | 101% | 103% |
| 11/25/08 | 52.8 | 4% | 75% | 102% | 104% |
| 11/26/08 | 52.7 | 4% | 74% | 101% | 103% |
| 11/27/08 | 51.9 | 4% | 73% | 101% | 102% |
| 11/28/08 | 52.3 | 4% | 73% | 101% | 102% |
| 11/29/08 | 52.5 | 5% | 73% | 101% | 103% |
| 11/30/08 | 52.2 | 4% | 73% | 102% | 104% |
| 12/01/08 | 52.8 | 4% | 73% | 102% | 104% |
| 12/02/08 | 51.3 | 4% | 76% | 102% | 104% |
| 12/03/08 | 50.9 | 5% | 76% | 101% | 103% |
| 12/04/08 | 49.6 | 5% | 78% | 104% | 105% |
| 12/05/08 | 47.8 | 5% | 80% | 104% | 105% |
| 12/06/08 | 46.3 | 5% | 81% | 103% | 105% |
| 12/07/08 | 46.0 | 5% | 81% | 103% | 105% |
| 12/08/08 | 46.5 | 6% | 80% | 102% | 103% |
| 12/09/08 | 46.5 | 5% | 80% | 102% | 104% |
| 12/10/08 | 47.0 | 5% | 82% | 104% | 106% |
| 12/11/08 | 46.3 | 5% | 82% | 104% | 107% |
| 12/12/08 | 46.0 | 5% | 82% | 103% | 105% |
| 12/13/08 | 46.3 | 5% | 82% | 102% | 104% |
| 12/14/08 | 45.6 | 6% | 87% | 102% | 103% |
| 12/15/08 | 42.6 | 8% | 99% | 103% | 105% |
| 12/16/08 | 42.8 | 8% | 105% | 104% | 105% |
| 12/17/08 | 45.0 | 8% | 102% | 103% | 103% |
| 12/18/08 | 48.9 | 5% | 81% | 101% | 102% |
| 12/19/08 | 49.5 | 5% | 74% | 101% | 103% |
| 12/20/08 | 49.7 | 5% | 72% | 101% | 103% |
| 12/21/08 | 48.1 | 4% | 73% | 103% | 105% |
| 12/22/08 | 49.7 | 4% | 74% | 103% | 106% |
| 12/23/08 | 51.2 | 4% | 73% | 101% | 103% |
| 12/24/08 | 51.2 | 4% | 72% | 100% | 103% |
| 12/25/08 | 50.6 | 5% | 75% | 101% | 103% |
| 12/26/08 | 50.3 | 6% | 80% | 101% | 102% |
| 12/27/08 | 50.1 | 5% | 80% | 101% | 103% |
| 12/28/08 | 49.9 | 5% | 81% | 103% | 104% |
| 12/29/08 | 51.2 | 6% | 82% | 102% | 103% |
| 12/30/08 | 46.0 | 5% | 82% | 98% | 100% |
| 12/31/08 | 50.8 | 6% | 78% | 102% | 104% |
| 01/01/09 | 49.9 | 5% | 82% | 102% | 104% |
| 01/02/09 | 50.0 | 5% | 82% | 102% | 103% |
| 01/03/09 | 50.5 | 5% | 82% | 102% | 103% |
| 01/04/09 | 50.1 | 5% | 82% | 101% | 102% |
| 01/05/09 | 50.0 | 5% | 81% | 101% | 103% |
| 01/06/09 | 50.0 | 5% | 82% | 102% | 104% |
| 01/07/09 | 49.9 | 5% | 82% | 103% | 104% |
| 01/08/09 | 49.8 | 5% | 82% | 102% | 103% |
| 01/09/09 | 54.0 | 3% | 73% | 101% | 102% |
| 01/10/09 | 54.7 | 4% | 71% | 101% | 103% |
| 01/11/09 | 52.0 | 5% | 79% | 103% | 105% |
| 01/12/09 | 45.8 | 6% | 94% | 105% | 105% |
| 01/13/09 | 54.4 | 4% | 75% | 102% | 105% |
| 01/14/09 | 54.5 | 4% | 74% | 102% | 103% |
| 01/15/09 | 54.2 | 4% | 74% | 103% | 105% |
| 01/16/09 | 55.1 | 4% | 73% | 103% | 105% |
| 01/17/09 | 55.8 | 4% | 71% | 101% | 104% |
| 01/18/09 | 55.4 | 4% | 71% | 101% | 103% |
| 01/19/09 | 55.7 | 4% | 72% | 102% | 103% |
| 01/20/09 | 55.8 | 4% | 70% | 102% | 105% |
| 01/21/09 | 55.5 | 4% | 69% | 101% | 103% |
| 01/22/09 | 55.8 | 4% | 70% | 101% | 103% |
| 01/23/09 | 56.1 | 4% | 69% | 101% | 102% |
| 01/24/09 | 56.5 | 4% | 69% | 101% | 103% |
| 01/25/09 | 56.6 | 4% | 69% | 101% | 103% |
| 01/26/09 | 56.9 | 4% | 69% | 100% | 102% |
| 01/27/09 | 56.7 | 4% | 70% | 100% | 102% |
| 01/28/09 | 57.1 | 3% | 69% | 101% | 104% |
| 01/29/09 | 56.9 | 4% | 68% | 102% | 104% |
| 01/30/09 | 57.2 | 4% | 68% | 101% | 103% |
| 01/31/09 | 56.9 | 4% | 67% | 101% | 103% |
| 02/01/09 | 56.8 | 4% | 67% | 100% | 103% |
| 02/02/09 | 55.2 | 4% | 67% | 102% | 104% |
| 02/03/09 | 55.1 | 4% | 66% | 101% | 103% |
| 02/04/09 | 54.0 | 4% | 67% | 102% | 105% |
| 02/05/09 | 54.3 | 4% | 70% | 101% | 103% |
| 02/06/09 | 54.6 | 4% | 66% | 100% | 103% |
| 02/07/09 | 54.6 | 4% | 64% | 100% | 102% |
| 02/08/09 | 54.4 | 4% | 63% | 100% | 103% |
| 02/09/09 | 54.7 | 4% | 63% | 100% | 102% |
| 02/10/09 | 54.8 | 4% | 62% | 99% | 102% |
| 02/11/09 | 54.6 | 4% | 62% | 100% | 102% |
| 02/12/09 | 55.0 | 4% | 62% | 100% | 102% |
| 02/13/09 | 55.0 | 3% | 61% | 100% | 102% |
| 02/14/09 | 55.0 | 4% | 62% | 100% | 103% |
| 02/15/09 | 55.1 | 4% | 61% | 101% | 103% |
| 02/16/09 | 55.2 | 4% | 61% | 100% | 103% |
| 02/17/09 | 55.2 | 4% | 61% | 100% | 102% |
| 02/18/09 | 55.3 | 4% | 60% | 100% | 102% |
| 02/19/09 | 55.0 | 4% | 60% | 101% | 104% |
| 02/20/09 | 55.2 | 3% | 60% | 100% | 103% |
| 02/21/09 | 55.2 | 4% | 59% | 100% | 103% |
| 02/22/09 | 55.2 | 4% | 59% | 101% | 104% |
| 02/23/09 | 55.1 | 4% | 59% | 100% | 104% |
| 02/24/09 | 55.5 | 4% | 58% | 99% | 103% |
| 02/25/09 | 55.4 | 3% | 57% | 99% | 101% |
| 02/26/09 | 55.1 | 3% | 57% | 99% | 102% |
| 02/27/09 | 53.0 | 4% | 60% | 100% | 102% |
| 02/28/09 | 52.6 | 4% | 61% | 101% | 105% |
| 03/01/09 | 52.5 | 4% | 58% | 101% | 105% |
| 03/01/09 | 52.5 | 4% | 58% | 101% | 105% |
| 03/02/09 | 55.0 | 3% | 57% | 100% | 104% |
| 03/03/09 | 54.4 | 4% | 56% | 100% | 103% |
| 03/04/09 | 56.0 | 4% | 58% | 99% | 102% |
| 03/05/09 | 55.7 | 4% | 59% | 100% | 102% |
| 03/06/09 | 56.6 | 4% | 57% | 99% | 102% |
| 03/07/09 | 56.3 | 4% | 56% | 99% | 102% |
| 03/08/09 | 56.3 | 3% | 56% | 98% | 102% |
| 03/09/09 | 56.0 | 4% | 55% | 99% | 102% |
| 03/10/09 | 56.4 | 4% | 54% | 99% | 102% |
| 03/11/09 | 56.4 | 4% | 54% | 98% | 102% |
| 03/12/09 | 56.3 | 4% | 54% | 99% | 103% |
| 03/13/09 | 55.6 | 3% | 53% | 99% | 103% |
| 03/14/09 | 55.9 | 4% | 53% | 99% | 103% |
| 03/15/09 | 55.6 | 3% | 53% | 99% | 102% |
| 03/16/09 | 56.0 | 3% | 53% | 99% | 103% |
| 03/17/09 | 55.9 | 4% | 53% | 99% | 102% |
| 03/18/09 | 55.9 | 3% | 53% | 99% | 102% |
| 03/19/09 | 55.6 | 3% | 52% | 98% | 102% |
| 03/20/09 | 55.5 | 4% | 52% | 99% | 103% |
| 03/21/09 | 56.2 | 4% | 52% | 98% | 102% |
| 03/22/09 | 56.6 | 3% | 53% | 98% | 102% |
| 03/23/09 | 56.7 | 4% | 52% | 98% | 102% |
| 03/24/09 | 56.4 | 4% | 52% | 98% | 101% |
| 03/25/09 | 57.3 | 3% | 50% | 98% | 101% |
| 03/26/09 | 45.8 | 8% | 55% | 101% | 105% |
| 03/27/09 | 56.3 | 3% | 53% | 99% | 102% |
| 03/28/09 | 56.6 | 3% | 52% | 98% | 104% |
| 03/29/09 | 56.1 | 4% | 53% | 100% | 103% |
| 03/30/09 | 56.4 | 3% | 52% | 99% | 102% |
| 03/31/09 | 57.3 | 3% | 47% | 97% | 102% |
| 04/01/09 | 57.0 | 3% | 48% | 98% | 102% |
| 04/02/09 | 56.9 | 3% | 48% | 97% | 102% |
| 04/03/09 | 56.3 | 3% | 49% | 98% | 102% |
| 04/04/09 | 57.1 | 3% | 47% | 96% | 101% |
| 04/05/09 | 57.1 | 2% | 46% | 97% | 102% |
| 04/06/09 | 56.9 | 3% | 47% | 98% | 104% |
| 04/07/09 | 57.0 | 2% | 47% | 98% | 103% |
| 04/08/09 | 57.4 | 3% | 48% | 98% | 102% |
| 04/09/09 | 57.1 | 4% | 50% | 99% | 102% |
| 04/10/09 | 57.3 | 3% | 47% | 97% | 101% |
| 04/11/09 | 57.2 | 4% | 47% | 98% | 103% |
| 04/12/09 | 57.1 | 3% | 47% | 98% | 102% |
| 04/13/09 | 57.1 | 3% | 46% | 97% | 102% |
| 04/14/09 | 57.1 | 4% | 47% | 98% | 103% |
| 04/15/09 | 57.0 | 4% | 46% | 98% | 102% |
| 04/16/09 | 57.1 | 4% | 46% | 98% | 102% |
| 04/17/09 | 57.3 | 3% | 46% | 98% | 102% |
| 04/18/09 | 57.3 | 3% | 46% | 97% | 102% |
| 04/19/09 | 57.1 | 3% | 45% | 97% | 102% |
| 04/20/09 | 56.9 | 4% | 46% | 97% | 103% |
| 04/21/09 | 57.0 | 3% | 45% | 97% | 102% |
| 04/22/09 | 57.0 | 3% | 45% | 97% | 101% |
| 04/23/09 | 57.0 | 3% | 45% | 97% | 101% |
| 04/24/09 | 57.2 | 3% | 45% | 97% | 102% |
| 04/25/09 | 57.2 | 4% | 45% | 97% | 102% |
| 04/26/09 | 57.2 | 3% | 44% | 96% | 101% |
| 04/27/09 | 57.4 | 4% | 45% | 97% | 101% |
| 04/28/09 | 57.4 | 3% | 44% | 97% | 102% |
| 04/29/09 | 57.5 | 4% | 44% | 97% | 101% |
| 04/30/09 | 57.0 | 3% | 44% | 96% | 101% |
| 05/01/09 | 57.2 | 3% | 44% | 97% | 101% |
| 05/02/09 | 57.5 | 3% | 45% | 97% | 101% |
| 05/03/09 | 57.1 | 3% | 45% | 98% | 102% |
| 05/04/09 | 57.3 | 3% | 44% | 97% | 102% |
| 05/05/09 | 57.3 | 3% | 43% | 96% | 101% |
| 05/06/09 | 57.5 | 3% | 43% | 97% | 101% |
| 05/07/09 | 57.9 | 3% | 43% | 97% | 101% |
| 05/08/09 | 57.8 | 3% | 43% | 96% | 101% |
| 05/09/09 | 57.9 | 3% | 43% | 96% | 101% |
| 05/10/09 | 57.8 | 3% | 43% | 97% | 101% |
| 05/11/09 | 57.8 | 3% | 42% | 96% | 101% |
| 05/12/09 | 57.9 | 3% | 42% | 96% | 101% |
| 05/13/09 | 58.1 | 3% | 42% | 96% | 101% |
| 05/14/09 | 57.9 | 4% | 43% | 96% | 101% |
| 05/15/09 | 57.5 | 3% | 43% | 96% | 101% |
| 05/16/09 | 57.1 | 3% | 42% | 96% | 102% |
| 05/17/09 | 57.0 | 3% | 42% | 97% | 102% |
| 05/18/09 | 56.9 | 2% | 41% | 97% | 102% |
| 05/19/09 | 56.8 | 2% | 41% | 97% | 102% |
| 05/20/09 | 57.2 | 2% | 41% | 96% | 101% |
| 05/21/09 | 57.0 | 2% | 41% | 96% | 102% |
| 05/22/09 | 57.0 | 2% | 40% | 96% | 102% |
| 05/23/09 | 57.1 | 3% | 40% | 95% | 101% |
| 05/24/09 | 57.2 | 2% | 39% | 95% | 101% |
| 05/25/09 | 57.0 | 2% | 39% | 95% | 101% |
| 05/26/09 | 57.0 | 2% | 40% | 95% | 101% |
| 05/27/09 | 57.2 | 3% | 40% | 96% | 101% |
| 05/28/09 | 57.2 | 3% | 40% | 95% | 101% |
| 05/29/09 | 57.2 | 3% | 40% | 96% | 102% |
| 05/30/09 | 57.8 | 3% | 39% | 95% | 101% |
| 05/31/09 | 57.3 | 2% | 39% | 95% | 101% |
| 06/01/09 | 57.6 | 2% | 39% | 95% | 101% |
| 06/02/09 | 57.6 | 2% | 39% | 95% | 101% |
| 06/03/09 | 57.4 | 3% | 38% | 95% | 101% |
| 06/04/09 | 57.1 | 3% | 38% | 95% | 102% |
| 06/05/09 | 56.7 | 3% | 40% | 96% | 102% |
| 06/06/09 | 56.8 | 3% | 41% | 96% | 101% |
| 06/07/09 | 57.1 | 3% | 38% | 95% | 101% |
| 06/07/09 | 57.1 | 3% | 38% | 95% | 101% |
| 06/08/09 | 57.2 | 2% | 38% | 95% | 100% |
| 06/09/09 | 57.2 | 2% | 37% | 94% | 100% |
| 06/10/09 | 57.3 | 3% | 37% | 95% | 100% |
| 06/11/09 | 57.1 | 2% | 37% | 95% | 100% |
| 06/12/09 | 57.2 | 2% | 37% | 95% | 100% |
| 06/13/09 | 57.1 | 2% | 37% | 95% | 100% |
| 06/14/09 | 57.4 | 2% | 36% | 94% | 100% |
| 06/15/09 | 57.2 | 2% | 36% | 95% | 100% |
| 06/16/09 | 57.4 | 3% | 36% | 94% | 100% |
| 06/17/09 | 57.2 | 2% | 36% | 94% | 101% |
| 06/18/09 | 57.1 | 2% | 36% | 94% | 101% |
| 06/19/09 | 57.2 | 3% | 36% | 94% | 101% |
| 06/20/09 | 57.4 | 3% | 36% | 94% | 100% |
| 06/21/09 | 57.1 | 2% | 36% | 94% | 100% |
| 06/22/09 | 56.9 | 2% | 35% | 94% | 101% |
| 06/23/09 | 57.1 | 2% | 35% | 94% | 100% |
| 06/24/09 | 56.8 | 3% | 35% | 94% | 101% |
| 06/25/09 | 56.9 | 3% | 35% | 94% | 100% |
| 06/26/09 | 57.0 | 3% | 35% | 94% | 101% |
| 06/27/09 | 56.9 | 2% | 34% | 94% | 100% |
| 06/28/09 | 56.8 | 2% | 34% | 94% | 101% |
| 06/29/09 | 57.1 | 3% | 34% | 94% | 101% |
| 06/30/09 | 56.9 | 2% | 34% | 94% | 100% |
| 07/01/09 | 56.9 | 3% | 34% | 94% | 101% |
| 07/02/09 | 57.0 | 2% | 34% | 93% | 100% |
| 07/03/09 | 57.1 | 2% | 34% | 93% | 100% |
| 07/04/09 | 56.9 | 2% | 33% | 93% | 100% |
| 07/05/09 | 56.8 | 2% | 33% | 93% | 100% |
| 07/06/09 | 57.0 | 2% | 33% | 94% | 101% |
| 07/07/09 | 57.0 | 3% | 33% | 94% | 101% |
| 07/08/09 | 56.8 | 2% | 33% | 93% | 101% |
| 07/09/09 | 50.4 | 3% | 46% | 98% | 102% |
| 07/10/09 | 49.7 | 3% | 50% | 100% | 102% |
| 07/11/09 | 53.1 | 3% | 37% | 95% | 101% |
| 07/12/09 | 57.0 | 2% | 31% | 92% | 100% |
| 07/13/09 | 56.7 | 2% | 30% | 92% | 100% |
| 07/14/09 | 56.9 | 2% | 30% | 92% | 100% |
| 07/15/09 | 57.1 | 3% | 30% | 92% | 100% |
| 07/16/09 | 57.1 | 3% | 30% | 93% | 100% |
| 07/17/09 | 56.9 | 2% | 30% | 92% | 101% |
| 07/18/09 | 57.2 | 2% | 30% | 92% | 101% |
| 07/19/09 | 57.0 | 2% | 30% | 92% | 101% |
| 07/20/09 | 56.9 | 2% | 30% | 92% | 101% |
| 07/21/09 | 57.0 | 3% | 30% | 93% | 101% |
| 07/22/09 | 57.0 | 3% | 30% | 93% | 100% |
| 07/23/09 | 56.8 | 3% | 30% | 93% | 101% |
| 07/24/09 | 56.7 | 3% | 30% | 92% | 101% |
| 07/25/09 | 56.8 | 3% | 29% | 92% | 100% |
| 07/26/09 | 56.9 | 3% | 29% | 92% | 101% |
| 07/27/09 | 56.2 | 3% | 30% | 93% | 101% |
| 07/28/09 | 54.4 | 2% | 31% | 94% | 101% |
| 07/29/09 | 56.9 | 3% | 29% | 92% | 100% |
| 07/30/09 | 57.0 | 2% | 28% | 92% | 100% |
| 07/31/09 | 56.8 | 2% | 29% | 91% | 100% |
| 08/01/09 | 57.3 | 3% | 29% | 92% | 100% |
| 08/02/09 | 57.5 | 2% | 28% | 91% | 100% |
| 08/03/09 | 57.5 | 3% | 28% | 91% | 100% |
| 08/04/09 | 57.4 | 2% | 28% | 91% | 100% |
| 08/05/09 | 56.5 | 2% | 28% | 91% | 101% |
| 08/06/09 | 56.9 | 2% | 28% | 91% | 101% |
| 08/07/09 | 57.2 | 2% | 28% | 91% | 101% |
| 08/08/09 | 57.2 | 2% | 28% | 91% | 100% |
| 08/09/09 | 57.3 | 3% | 28% | 90% | 100% |
| 08/10/09 | 57.1 | 2% | 27% | 90% | 100% |
| 08/11/09 | 57.3 | 2% | 26% | 90% | 100% |
| 08/12/09 | 57.4 | 2% | 28% | 91% | 100% |
| 08/13/09 | 57.3 | 3% | 31% | 93% | 101% |
| 08/14/09 | 57.2 | 2% | 27% | 91% | 101% |
| 08/15/09 | 57.8 | 3% | 26% | 90% | 100% |
| 08/16/09 | 57.6 | 2% | 26% | 90% | 100% |
| 08/17/09 | 57.5 | 3% | 27% | 91% | 100% |
| 08/18/09 | 57.3 | 3% | 26% | 90% | 100% |
| 08/19/09 | 57.4 | 3% | 26% | 90% | 100% |
| 08/20/09 | 57.5 | 2% | 25% | 89% | 100% |
| 08/21/09 | 56.5 | 2% | 26% | 91% | 101% |
| 08/22/09 | 57.6 | 3% | 26% | 90% | 101% |
| 08/23/09 | 57.4 | 2% | 26% | 91% | 101% |
| 08/24/09 | 57.7 | 3% | 25% | 90% | 101% |
| 08/25/09 | 57.6 | 3% | 24% | 89% | 100% |
| 08/26/09 | 57.8 | 3% | 24% | 88% | 100% |
| 08/27/09 | 57.6 | 2% | 24% | 88% | 100% |
| 08/28/09 | 57.7 | 3% | 24% | 88% | 100% |
| 08/29/09 | 57.9 | 3% | 23% | 88% | 100% |
| 08/30/09 | 57.7 | 3% | 24% | 88% | 101% |
| 08/31/09 | 55.0 | 3% | 24% | 88% | 100% |
| 09/01/09 | 57.6 | 2% | 25% | 90% | 102% |
| 09/02/09 | 57.8 | 3% | 23% | 88% | 101% |
| 09/03/09 | 57.7 | 3% | 23% | 88% | 101% |
| 09/04/09 | 57.4 | 2% | 23% | 88% | 100% |
| 09/05/09 | 57.2 | 2% | 23% | 88% | 100% |
| 09/06/09 | 57.3 | 2% | 23% | 88% | 100% |
| 09/07/09 | 57.3 | 2% | 23% | 87% | 100% |
| 09/08/09 | 57.4 | 3% | 22% | 87% | 100% |
| 09/09/09 | 57.4 | 3% | 22% | 86% | 100% |
| 09/10/09 | 57.3 | 2% | 21% | 86% | 100% |
| 09/11/09 | 57.4 | 3% | 21% | 86% | 100% |
| 09/12/09 | 57.5 | 3% | 21% | 86% | 100% |
| 09/13/09 | 57.5 | 3% | 21% | 85% | 100% |

Various terms are used herein, to the extent a term used in not defined herein, it should be given the broadest definition persons in the pertinent art have given that term as reflected in printed publications and issued patents.

The term "activity" refers to the weight of product produced per weight of the catalyst used in a process per hour of reaction at a standard set of conditions (e.g., grams product/gram catalyst/hr).

The term "alkyl" refers to a functional group or side-chain that consists solely of single-bonded carbon and hydrogen atoms, for example a methyl or ethyl group.

The term "alkylation" refers to the addition of an alkyl group to another molecule.

The term "conversion" refers to the percentage of input converted.

The term "deactivated catalyst" refers to a catalyst that has lost enough catalyst activity to no longer be efficient in a specified process.

The term "high poison feed stream" refers to a feed stream that typically contains impurities that deactivate a catalyst in quantities that range from 10 ppb to 100 ppb or more and can typically average from 20 ppb to 40 ppb.

The term "molecular sieve" refers to a material having a fixed, open-network structure, usually crystalline, that may be used to separate hydrocarbons or other mixtures by selective occlusion of one or more of the constituents, or may be used as a catalyst in a catalytic conversion process.

The term "recycle" refers to returning an output of a system as input to either that same system or another system within a process. The output may be recycled to the system in any manner known to one skilled in the art, for example, by combining the output with the input stream or by directly feeding the output into the system. In addition, multiple input streams may be fed to a system in any manner known to one skilled in the art.

The term "regenerated catalyst" refers to a catalyst that has regained enough activity to be efficient in a specified process. Such efficiency is determined by individual process parameters.

The term "regeneration" refers to a process for renewing catalyst activity and/or making a catalyst reusable after its activity has reached an unacceptable level. Examples of such regeneration may include passing steam over a catalyst bed or burning off carbon residue, for example.

The term "transalkylation" refers to the transfer of an alkyl group from one aromatic molecule to another.

The term "zeolite" refers to a molecular sieve containing a silicate lattice, usually in association with some aluminum, boron, gallium, iron, and/or titanium, for example. In the following discussion and throughout this disclosure, the terms molecular sieve and zeolite will be used more or less interchangeably. One skilled in the art will recognize that the teachings relating to zeolites are also applicable to the more general class of materials called molecular sieves.

## Claims

1. A method of producing an alkylaromatic by the alkylation of an aromatic with an alkylating agent, the method comprising:
providing at least one reaction zone containing a H-beta zeolite catalyst, wherein the at least one reaction zone comprises at least one preliminary alkylation reactor and at least one primary alkylation reactor downstream of the preliminary alkylation reactor, wherein the H-beta zeolite catalyst is contained in the preliminary alkylation reactor and a catalyst other than the H-beta zeolite catalyst is contained in the primary alkylation reactor;
introducing a feed stream comprising an aromatic and an alkylating agent to the reaction zone, wherein the feed stream further includes catalyst poisons, which are nitrogen compounds; and
reacting at least a portion of the aromatic under alkylation conditions to produce an alkylaromatic.

2. The process of claim 1, wherein the feed stream further comprises said catalyst poisons averaging at least 5 ppb.

3. The process of claim 1, wherein the feed stream further comprises said catalyst poisons averaging at least 30 ppb.

4. The process of claim 1, wherein the feed stream further comprises said catalyst poisons averaging at least 75 ppb.

5. The method of claim 1, wherein the amount of H-beta catalyst in the at least one reaction zone is at least 1360 kg (3,000 pounds).

6. The method of claim 1, wherein the amount of H-beta catalyst in the at least one reaction zone is between 1360 kg (3,000 pounds) and 22,700 kg (50,000 pounds) in said first preliminary alkylation system.

7. The method of claim 1, wherein the alkylaromatic production is at least 0.5 million pounds per day.

8. The method of claim 1, wherein the alkylaromatic is ethylbenzene, the aromatic is benzene and the alkylating agent is ethylene.

9. The method of claim 1, wherein the first preliminary alkylation system has a run time of at least 6 months prior to regeneration.

10. The method of claim 1, wherein the first preliminary alkylation system has a run time of at least 12 months prior to regeneration.

11. The method of claim 1, wherein the first preliminary alkylation system has a run time of at least 18 months prior to regeneration.

12. The method of claim 1, wherein the H-beta zeolite catalyst in the first preliminary alkylation system can be regenerated in-situ.

13. The method of claim 1, wherein the first preliminary alkylation system can be bypassed for catalyst regeneration without taking the at least one primary alkylation reactor out of service.

14. The method of claim 6, wherein the primary alkylation reactor experiences a decrease in catalyst deactivation when the preliminary alkylation reactor is in service.

15. The method of claim 6, wherein the primary alkylation reactor experiences no catalyst deactivation when the preliminary alkylation reactor is in service.

16. The method of claim 1 which is a process of producing ethylbenzene from a high poison feed stream by the alkylation of benzene with ethylene, the process comprising:
introducing a feed stream comprising benzene and ethylene to the reaction zone, the feed stream containing at least 10 ppb poisons; and
reacting at least a portion of the benzene with ethylene under alkylation conditions to produce ethylbenzene.

17. The method of claim 16, wherein the H-beta catalyst has a deactivation rate of no more than 13.6 kg (30 pounds) of catalyst per million pounds of ethylbenzene produced.

18. The method of claim 16, wherein the H-beta catalyst has a deactivation rate of no more than 4.54 kg (10 pounds) of catalyst per million pounds of ethylbenzene produced.

19. The method of claim 16, wherein the H-beta catalyst has a deactivation rate of no more than 1.13 kg (2.5 pounds) of catalyst per million pounds of ethylbenzene produced.

20. The method of claim 16, wherein the amount of catalyst in the at least one reaction zone is from 1360 to 3180 kg (3,000 to 7,000 pounds) and produces over 454 million kg (1,000 million pounds) of ethylbenzene prior to regeneration of the catalyst.

21. The method of claim 16, wherein the amount of catalyst in the at least one reaction zone is from 4540 to 5440 kg (10,000 to 12,000 pounds) and produces over 908 million kg (2,000 million pounds) of ethylbenzene prior to regeneration of the catalyst.

22. The method of claim 16, wherein the amount of catalyst in the at least one reaction zone is from 9070 to 9980 kg (20,000 to 22,000 pounds) and produces over 1816 million kg (4,000 million pounds) of ethylbenzene prior to regeneration of the catalyst.

23. The method of claim 1 which is a process of producing ethylbenzene from a high poison feed stream by the alkylation of benzene with ethylene, the process comprising:
providing said at least one reaction zone, which contains 9070 to 1130 kg (20,000 to 25,000 pounds) of H-beta zeolite catalyst;
introducing a high poison feed stream comprising benzene containing at least 10 ppb poisons and ethylene to the at least one reaction zone in amounts averaging at least 7.26 million kg (16 million pounds) benzene per day;
reacting at least a portion of the benzene with ethylene under alkylation conditions to produce ethylbenzene averaging at least 3.18 million kg (7.0 million pounds) per day, for a cumulative run time of 6 months to produce a total of at least 544 million kg (1,200 million pounds) of ethylbenzene;
wherein the catalyst deactivation is less than 20% of the total H-beta zeolite catalyst charge at the end of the run.

## Patentansprüche

1. Verfahren zum Herstellen eines Alkylaromastoffs durch Alkylieren eines Aromastoffs mit einem Alkylierungsmittel, wobei das Verfahren umfasst:
Bereitstellen zumindest einer Reaktionszone, die einen H-beta-Zeolith-Katalysator enthält, wobei die zumindest eine Reaktionszone zumindest einen Voralkylierungsreaktor und zumindest einen primären Alkylierungsreaktor umfasst, der dem Voralkylierungsreaktor nachgeschaltet ist, wobei der H-beta-Zeolith-Katalysator im Voralkylierungsreaktor enthalten ist und ein Katalysator, bei dem es sich nicht um den H-beta-Zeolith-Katalysator handelt, ist im primären Alkylierungsreaktor enthalten ist;
Einbringen eines Zustroms, der einen Aromastoff und ein Alkylierungsmittel umfasst, in die Reaktionszone, wobei der Zustrom des Weiteren Katalysatorgifte beinhaltet, die Stickstoffverbindungen sind; und
Reagieren zumindest eines Teils des Aromastoffs unter Alkylierungsbedingungen, um einen Alkylaromastoff herzustellen.

2. Prozess nach Anspruch 1, wobei der Zustrom des Weiteren die Katalysatorgifte in einem Durchschnitt von zumindest 5 ppb umfasst.

3. Prozess nach Anspruch 1, wobei der Zustrom des Weiteren die Katalysatorgifte in einem Durchschnitt von zumindest 30 ppb umfasst.

4. Prozess nach Anspruch 1, wobei der Zustrom des Weiteren die Katalysatorgifte in einem Durchschnitt von zumindest 75 ppb umfasst.

5. Verfahren nach Anspruch 1, wobei die Menge an H-beta-Katalysator in der zumindest einen Reaktionszone zumindest 1360 kg (3000 Pfund) beträgt.

6. Verfahren nach Anspruch 1, wobei die Menge an H-beta-Katalysator in der zumindest einen Reaktionszone im ersten Voralkylierungssystem zwischen 1360 kg (3000 Pfund) und 22.700 kg (50.000 Pfund) beträgt.

7. Verfahren nach Anspruch 1, wobei die Alkylaromastoffherstellung zumindest 0,5 Millionen Pfund pro Tag beträgt.

8. Verfahren nach Anspruch 1, wobei der Alkylaromastoff Ethylbenzol ist, wobei der Aromastoff Benzol ist und das Alkylierungsmittel Ethylen ist.

9. Verfahren nach Anspruch 1, wobei das erste Voralkylierungssystem eine Laufzeit von zumindest 6 Monaten vor Regeneration aufweist.

10. Verfahren nach Anspruch 1, wobei das erste Voralkylierungssystem eine Laufzeit von zumindest 12 Monaten vor Regeneration aufweist.

11. Verfahren nach Anspruch 1, wobei das erste Voralkylierungssystem eine Laufzeit von zumindest 18 Monaten vor Regeneration aufweist.

12. Verfahren nach Anspruch 1, wobei der H-beta-Zeolith-Katalysator im ersten Voralkylierungssystem in situ regeneriert werden kann.

13. Verfahren nach Anspruch 1, wobei das erste Voralkylierungssystem in Bezug auf eine Katalysatorregeneration umgangen werden kann, ohne dass der zumindest eine primäre Alkylierungsreaktor außer Betrieb genommen wird.

14. Verfahren nach Anspruch 6, wobei der primäre Alkylierungsreaktor eine Verringerung der Katalysatordeaktivierung erfährt, wenn der Voralkylierungsreaktor in Betrieb ist.

15. Verfahren nach Anspruch 6, wobei der primäre Alkylierungsreaktor keine Verringerung der Katalysatordeaktivierung erfährt, wenn der Voralkylierungsreaktor in Betrieb ist.

16. Verfahren nach Anspruch 1, bei dem es sich um einen Prozess des Herstellens von Ethylbenzol aus einem Zustrom mit hohem Giftgehalt durch Alkylierung von Benzol mit Ethylen handelt, wobei der Prozess umfasst:
Einbringen eines Zustroms, der Benzol und Ethylen umfasst, in die Reaktionszone, wobei der Zustrom zumindest 10 ppb Gifte enthält; und
Reagieren zumindest eines Teils des Benzols mit Ethylen unter Alkylierungsbedingungen, um Ethylbenzol herzustellen.

17. Verfahren nach Anspruch 16, wobei der H-beta-Katalysator eine Deaktivierungsrate von nicht mehr als 13,6 kg (30 Pfund) Katalysator pro Millionen Pfund hergestellten Ethylbenzols aufweist.

18. Verfahren nach Anspruch 16, wobei der H-beta-Katalysator eine Deaktivierungsrate von nicht mehr als 4,54 (10 Pfund) Katalysator pro Millionen Pfund hergestellten Ethylbenzols aufweist.

19. Verfahren nach Anspruch 16, wobei der H-beta-Katalysator eine Deaktivierungsrate von nicht mehr als 1,13 kg (2,5 Pfund) Katalysator pro Millionen Pfund hergestellten Ethylbenzols aufweist.

20. Verfahren nach Anspruch 16, wobei die Menge an Katalysator in der zumindest einen Reaktionszone 1360 bis 3180 kg (3000 bis 7000 Pfund) beträgt und mehr als 454 Millionen kg (1000 Millionen Pfund) Ethylbenzol vor Regeneration des Katalysators herstellt.

21. Verfahren nach Anspruch 16, wobei die Menge an Katalysator in der zumindest einen Reaktionszone 4540 bis 5440 kg (10.000 bis 12.000 Pfund) beträgt und mehr als 908 Millionen kg (2000 Millionen Pfund) Ethylbenzol vor Regeneration des Katalysators herstellt.

22. Verfahren nach Anspruch 16, wobei die Menge an Katalysator in der zumindest einen Reaktionszone 9070 bis 9980 kg (20.000 bis 22.000 Pfund) beträgt und mehr als 1816 Millionen kg (4000 Millionen Pfund) Ethylbenzol vor Regeneration des Katalysators herstellt.

23. Verfahren nach Anspruch 1, bei dem es sich um einen Prozess zum Herstellen von Ethylbenzol aus einem Zustrom mit hohem Giftgehalt durch Alkylierung von Benzol mit Ethylen handelt, wobei der Prozess umfasst:
Bereitstellen der zumindest einen Reaktionszone, die 9070 bis 1130 kg (20.000 bis 25.000 Pfund) H-beta-Zeolith-Katalysator enthält;
Einbringen eines Zustroms mit hohem Giftgehalt, der Benzol, enthaltend zumindest 10 pp Gifte, und Ethylen umfasst, zu der zumindest einen Reaktionszone in Mengen in einem Durchschnitt von zumindest 7,26 Millionen kg (16 Millionen Pfund) Benzol pro Tag;
Reagieren zumindest eines Teils des Benzols mit Ethylen unter Alkylierungsbedingungen, um Ethylbenzol in einem Durchschnitt von zumindest 3,18 Millionen kg (7,0 Millionen Pfund) pro Tag für eine kumulative Laufzeit von 6 Monaten herzustellen, um insgesamt zumindest 544 Millionen kg (1,200 Millionen Pfund) Ethylbenzol herzustellen;
wobei die Katalysatordeaktivierung weniger als 20 % der H-beta-Zeolith-Katalysator-Gesamtladung am Ende des Durchlaufs beträgt.

## Revendications

1. Procédé de production d'un composé alkylaromatique par alkylation d'un composé aromatique avec un agent d'alkylation, le procédé comprenant les étapes consistant à :
utiliser au moins une zone réactionnelle contenant un catalyseur zéolitique H-bêta, l'au moins une zone réactionnelle comprenant au moins un réacteur d'alkylation préliminaire et au moins un réacteur d'alkylation principal en aval du réacteur d'alkylation préliminaire, le catalyseur zéolitique H-bêta étant contenu dans le réacteur d'alkylation préliminaire et un catalyseur autre que le catalyseur zéolitique H-bêta étant contenu dans le réacteur d'alkylation principal ;
introduire un courant d'alimentation comprenant un composé aromatique et un agent d'alkylation dans la zone réactionnelle, le courant d'alimentation comprenant en outre des poisons catalytiques qui sont des composés azotés ; et
faire réagir au moins une partie du composé aromatique dans des conditions d'alkylation pour produire un composé alkylaromatique.

2. Procédé selon la revendication 1, dans lequel le courant d'alimentation comprend en outre lesdits poisons catalytiques représentant une moyenne d'au moins 5 ppb.

3. Procédé selon la revendication 1, dans lequel le courant d'alimentation comprend en outre lesdits poisons catalytiques représentant une moyenne d'au moins 30 ppb.

4. Procédé selon la revendication 1, dans lequel le courant d'alimentation comprend en outre lesdits poisons catalytiques représentant une moyenne d'au moins 75 ppb.

5. Procédé selon la revendication 1, dans lequel la quantité du catalyseur H-bêta dans l'au moins une zone réactionnelle est d'au moins 1360 kg (3000 livres).

6. Procédé selon la revendication 1, dans lequel la quantité du catalyseur H-bêta dans l'au moins une zone réactionnelle est comprise entre 1360 kg (3000 livres) et 22 700 kg (50 000 livres) dans ledit premier système d'alkylation préliminaire.

7. Procédé selon la revendication 1, dans lequel la production du composé alkylaromatique est d'au moins 0,5 million de livres par jour.

8. Procédé selon la revendication 1, dans lequel le composé alkylaromatique est l'éthylbenzène, le composé aromatique est le benzène et l'agent d'alkylation est l'éthylène.

9. Procédé selon la revendication 1, dans lequel le premier système d'alkylation préliminaire a un temps d'exécution d'au moins 6 mois avant la régénération.

10. Procédé selon la revendication 1, dans lequel le premier système d'alkylation préliminaire a un temps d'exécution d'au moins 12 mois avant la régénération.

11. Procédé selon la revendication 1, dans lequel le premier système d'alkylation préliminaire a un temps d'exécution d'au moins 18 mois avant la régénération.

12. Procédé selon la revendication 1, dans lequel le catalyseur zéolitique H-bêta dans le premier système d'alkylation préliminaire peut être régénéré in-situ.

13. Procédé selon la revendication 1, dans lequel le premier système d'alkylation préliminaire peut être contourné pour la régénération du catalyseur sans mettre l'au moins un réacteur d'alkylation principal hors service.

14. Procédé selon la revendication 6, dans lequel le réacteur d'alkylation principal subit une diminution de la désactivation du catalyseur lorsque le réacteur d'alkylation préliminaire est en service.

15. Procédé selon la revendication 6, dans lequel le réacteur d'alkylation principal ne subit pas de diminution de la désactivation du catalyseur lorsque le réacteur d'alkylation préliminaire est en service.

16. Procédé selon la revendication 1 qui est un procédé de production d'éthylbenzène à partir d'un courant d'alimentation riche en poisons par alkylation de benzène avec de l'éthylène, le procédé comprenant les étapes consistant à :
introduire un courant d'alimentation comprenant du benzène et de l'éthylène dans la zone réactionnelle, le courant d'alimentation contenant au moins 10 ppb de poisons ; et
faire réagir au moins une partie du benzène avec l'éthylène dans des conditions d'alkylation pour produire l'éthylbenzène.

17. Procédé selon la revendication 16, dans lequel le catalyseur H-bêta a un taux de désactivation de pas plus de 13,6 kg (30 livres) de catalyseur par million de livres d'éthylbenzène produit.

18. Procédé selon la revendication 16, dans lequel le catalyseur H-bêta a un taux de désactivation de pas plus de 4,54 kg (10 livres) de catalyseur par million de livres d'éthylbenzène produit.

19. Procédé selon la revendication 16, dans lequel le catalyseur H-bêta a un taux de désactivation de pas plus de 1,13 kg (2,5 livres) de catalyseur par million de livres d'éthylbenzène produit.

20. Procédé selon la revendication 16, dans lequel la quantité de catalyseur dans l'au moins une zone réactionnelle est de 1360 à 3180 kg (3000 à 7000 livres) et produit plus de 454 millions de kg (1000 millions de livres) d'éthylbenzène avant la régénération du catalyseur.

21. Procédé selon la revendication 16, dans lequel la quantité de catalyseur dans l'au moins une zone réactionnelle est de 4540 à 5440 kg (10 000 à 12 000 livres) et produit plus de 908 millions de kg (2000 millions de livres) d'éthylbenzène avant la régénération du catalyseur.

22. Procédé selon la revendication 16, dans lequel la quantité de catalyseur dans l'au moins une zone réactionnelle est de 9070 à 9980 kg (20 000 à 22 000 livres) et produit plus de 1816 millions de kg (4000 millions de livres) d'éthylbenzène avant la régénération du catalyseur.

23. Procédé selon la revendication 1 qui est un procédé de production d'éthylbenzène à partir d'un courant d'alimentation riche en poisons par alkylation de benzène avec de l'éthylène, le procédé comprenant les étapes consistant à :
utiliser ladite au moins une zone réactionnelle, qui contient de 9070 à 1130 kg (20 000 à 25 000 livres) de catalyseur zéolitique H-bêta ;
introduire un courant d'alimentation riche en poisonscomprenant du benzène contenant au moins 10 ppb de poisons et de l'éthylène dans l'au moins une zone réactionnelle en des quantités représentant une moyenne d'au moins 7,26 millions de kg (16 millions de livres) de benzène par jour ;
faire réagir au moins une partie du benzène avec l'éthylène dans des conditions d'alkylation pour produire l'éthylbenzène représentant une moyenne d'au moins 3,18 millions de kg (7,0 millions de livres) par jour, pendant un temps d'exécution cumulé de 6 mois pour produire un total d'au moins 544 millions de kg (1200 millions de livres) d'éthylbenzène ;
dans lequel la désactivation du catalyseur est inférieure à 20 % de la charge de catalyseur zéolitique H-bêta totale à la fin de l'exécution.
